# EUROPÄISCHE PATENTSCHRIFT

(11) **EP 1 567 526 B1**
(45) Veröffentlichungstag und Bekanntmachung des Hinweises auf die Patenterteilung: **16.04.2008**
(21) Anmeldenummer: 03782212.9
(22) Anmeldetag: 19.11.2003
(51) Int. Cl.: C07D 451/06, A61K 31/46, A61P 11/06, A61P 13/10

(54) **CARBAMINSÄUREESTER MIT ANTICHOLINERGER WIRKSAMKEIT**
CARBAMIC ACID ESTERS WITH ANTICHOLINERGIC ACTION
ESTERS D'ACIDE CARBAMIQUE A ACTION ANTICHOLINERGIQUE

(30) Priorität: 26.11.2002 DE 10255040
(43) Veröffentlichungstag der Anmeldung: 31.08.2005
(73) Patentinhaber: Boehringer Ingelheim Pharma GmbH & Co.KG, 55216 Ingelheim am Rhein (DE)
(72) Erfinder: GRAUERT, Matthias, 88400 BIBERACH (DE); HOFFMANN, Matthias, 88441 MITTELBIBERACH (DE); PIEPER, Michael, P., 88400 BIBERACH (DE); SPECK, Georg, 55218 INGELHEIM (DE); BREITFELDER, Steffen, 88433 AssMANNSHARDT (DE)
(86) Internationale Anmeldenummer: PCT/EP2003/012912
(87) Internationale Veröffentlichungsnummer: WO 2004/048373

(56) Entgegenhaltungen:
- WO-A-97/34892
- US-A- 3 740 405
- DATABASE CAPLUS [Online] CHEMICAL ABSTRACTS SERVICE, COLUMBUS, OHIO, US; 12. Oktober 1995 (1995-10-12) Database accession no. 1995:849168 XP002272573 & WO 95 06635 A (YAMANOUCHI PHARMA CO LTD ; TAKEUCHI MAKOTO (JP); NAITO RYO (JP); MORIH) 9. März 1995 (1995-03-09)

## Beschreibung

Die vorliegende Erfindung betrifft neue Carbaminsäureester der allgemeinen Formel **1** 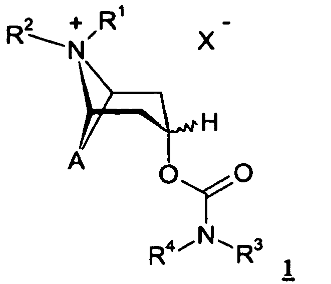 worin X - und die Gruppen A, R¹, R², R³ und R⁴ die in den Ansprüchen und in der Beschreibung genannten Bedeutungen haben können, Verfahren zu deren Herstellung sowie deren Verwendung als Arzneimittel, insbesondere als Arzneimittel mit anticholinerger Wirksamkeit.

### Beschreibung der Erfindung

Die vorliegende Erfindung betrifft Verbindungen der allgemeinen Formel **1** 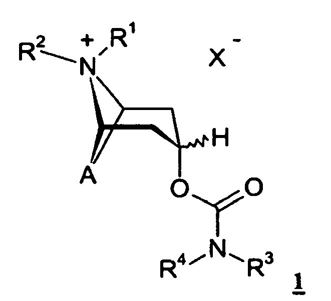 worin
- A: ein zweibindiger Rest ausgewählt aus der Gruppe bestehend aus 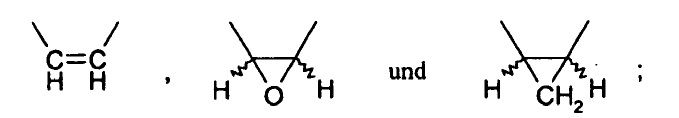
- X⁻: ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat;
- R¹ und R²: gleich oder verschieden, C₁-C₅-Alkyl, welches gegebenenfalls durch einen Rest ausgewählt aus der Gruppe bestehend aus -C₃-C₆-Cycloalkyl, Hydroxy oder Halogen substituiert sein kann,
oder
R¹ und R² gemeinsam eine C₃-C₅-Alkylen-Brücke;
- R³ und R⁴: gleich oder verschieden,
Wasserstoff, oder
C₁-C₅-Alkyl, welches gegebenenfalls ein- oder mehrfach substituiert sein kann durch einen oder mehrere Reste ausgewählt aus der Gruppe bestehend aus Hydroxy, Halogen, CF₃ und -OC₁-C₄-Alkyl, oder
eine C₂-C₅-Alkenyl- oder C₂-C₅-Alkinyl-Gruppe, welche gegebenenfalls ein- oder mehrfach substituiert sein kann durch einen oder mehrere Reste ausgewählt aus der Gruppe bestehend aus Hydroxy, Halogen, CF₃, -OC₁-C₄-Alkyl, Phenyl und Phenyl welches ein- oder mehrfach durch Methyl, Halogen, Hydroxy, CF₃ oder Methoxy substituiert sein kann, oder
C₆-C₁₀-Aryl, welches gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl welches ein- oder mehrfach durch Methyl, Halogen, Hydroxy, CF₃ oder Methoxy substituiert sein kann, oder
C₆-C₁₀-Aryl, welches durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder C₆-C₁₀-Aryl-C₁-C₄-alkylen, welches am Arylrest gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl welches ein- oder mehrfach durch Methyl, Halogen, Hydroxy, CF₃ oder Methoxy substituiert sein kann, oder
C₆-C₁₀-Aryl-C₁-C₄-alkylen, welches am Arylrest durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
C₆-C₁₀-Aryl-C₁-C₄-alkylen, welches an der Alkylengruppe gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl und Phenyl, oder
ein 5 oder 6-gliedriger gesättigter oder ungesättigter Ring, der ein, zwei oder drei Heteroatome ausgewählt aus der Gruppe bestehend aus Stickstoff, Sauerstoff oder Schwefel enthalten kann und der gegebenenfalls ein- oder mehrfach substituiert sein kann durch einen oder mehrere Reste ausgewählt aus der Gruppe bestehend aus C₁-C₄-Alkyl Hydroxy, Halogen, CF₃, Phenyl, Benzyl und -OC₁-C₄-Alkyl, oder
ein 5 oder 6-gliedriger gesättigter oder ungesättigter Ring, der ein, zwei oder drei Heteroatome ausgewählt aus der Gruppe bestehend aus Stickstoff, Sauerstoff oder Schwefel enthalten kann und der durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
C₃-C₆-Cycloalkyl, welches gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl welches ein- oder mehrfach durch Methyl, Halogen, Hydroxy, CF₃ oder Methoxy substituiert sein kann, oder
C₃-C₆-Cycloalkyl, das durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
ein Rest der Formel 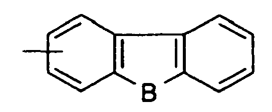 worin B -CH₂-, -NH-, -S- oder -O- bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 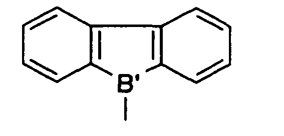 worin B' CH oder N bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 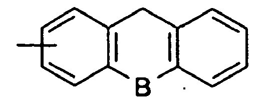 worin B -CH₂-, -NH-, -S- oder -O- bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 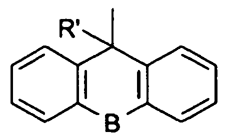 worin B -CH₂-, -NH-, -S- oder -O- bedeutet, R' für Wasserstoff, Hydroxy, Methyl, Hydroxymethyl, Ethyl, -CF₃, CHF₂ oder Halogen stehen kann, und der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
- R³ und R⁴: bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann und der gegebenenfalls ein- oder mehrfach substituiert sein kann durch einen oder mehrere Reste ausgewählt aus der Gruppe bestehend aus C₁-C₄-Alkyl Hydroxy, Halogen, CF₃, Phenyl, Benzyl und -OC₁-C₄-Alkyl, oder
- R³ und R⁴: bilden gemeinsam mit dem Stickstoffatom einen 5- oder 6-gliedrigen gesättigten oder ungesättigten heterocyclischen Ring, der durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Bevorzugt sind Verbindungen der allgemeinen Formel **1**, worin
- A: ein zweibindiger Rest ausgewählt aus der Gruppe bestehend aus 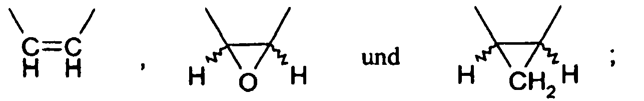
- X⁻: ein einfach negativ geladenes Anion, vorzugsweise ein Anion ausgewählt aus der Gruppe bestehend aus Chlorid, Bromid, Methansulfonat und p-Toluolsulfonat, bevorzugt Bromid;
- R¹ und R²: gleich oder verschieden, C₁-C₃-Alkyl, welches gegebenenfalls durch einen Rest ausgewählt aus der Gruppe bestehend aus C₃-C₅-Cycloalkyl, Hydroxy, oder Fluor substituiert sein kann,
oder
R¹ und R² gemeinsam eine C₃-C₄-Alkylen-Brücke;
- R³ und R⁴: gleich oder verschieden,
Wasserstoff, oder
C₁-C₅-Alkyl, welches gegebenenfalls substituiert sein kann durch einen Rest ausgewählt aus der Gruppe bestehend aus Hydroxy, Fluor, CF₃ und Methoxy, oder
ein Phenyl- oder Naphthylrest, welcher gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Phenyl- oder Naphthylrest, welcher durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Benzyl- oder Phenylethylrest, welcher am Phenylring gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Benzyl- oder Phenylethylrest, welcher am Phenylring durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Benzyl- oder Phenylethylrest, welcher an der Alkylenbrücke gegebenenfalls substituiert sein kann durch einen oder zwei, bevorzugt einen Rest ausgewählt aus der Gruppe bestehend aus Methyl, Ethyl, Hydroxy, Fluor, Chlor, Brom, CF₃, Methoxy und Phenyl, oder
ein 5, oder 6- gliedriger gesättigter oder ungesättigter Ring, der ein, zwei oder drei Heteroatome ausgewählt aus der Gruppe bestehend aus Stickstoff, Sauerstoff oder Schwefel enthalten kann und der gegebenenfalls ein-, zwei- oder dreifach substituiert sein kann durch einen oder mehrere Reste ausgewählt aus der Gruppe bestehend aus Methyl, Ethyl, Hydroxy, Fluor, Chlor, Brom, CF₃, Phenyl, Benzyl und Methoxy, oder
ein 5, oder 6- gliedriger gesättigter oder ungesättigter Ring, der ein, zwei oder drei Heteroatome ausgewählt aus der Gruppe bestehend aus Stickstoff, Sauerstoff oder Schwefel enthalten kann und der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Cyclopentyl- oder Cyclohexylrest, welcher gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Cyclopentyl- oder Cyclohexylrest, der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Rest der Formel 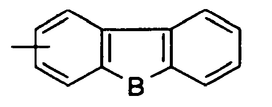 worin B -CH₂-, -NH-, -S- oder -O- bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 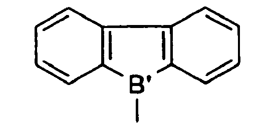 worin B' CH oder N bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 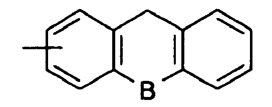 worin B -CH₂-, -NH-, -S- oder -O- bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 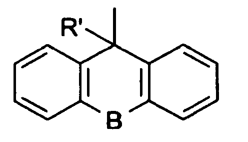 worin B -CH₂-, -NH-, -S- oder -O- bedeutet, R' für Wasserstoff, Hydroxy, Methyl, Hydroxymethyl, Ethyl, -CF₃, CHF₂ oder Fluor stehen kann, und der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
- R³ und R⁴: bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann und der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, Phenyl, CF₃ oder Methoxy, oder
- R³ und R⁴: bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann, der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Besonders bevorzugt sind Verbindungen der allgemeinen Formel 1, worin
- A: ein zweibindiger Rest ausgewählt aus der Gruppe bestehend aus 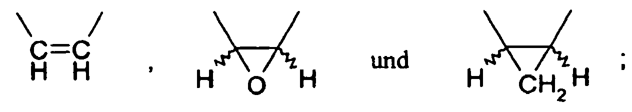
- X⁻: ein einfach negativ geladenes Anion, vorzugsweise ein Anion ausgewählt aus der Gruppe bestehend aus Chlorid, Bromid, Methansulfonat und p-Toluolsulfonat, bevorzugt Bromid;
- R¹ und R²: gleich oder verschieden, ein Methyl- oder Ethylrest, der gegebenenfalls durch Cyclopropyl, Hydroxy oder Fluor substituiert sein kann,
oder
R¹ und R² gemeinsam eine C₃-C₄-Alkylen-Brücke;
- R³: Wasserstoff oder C₁-C₃-Alkyl, welches gegebenenfalls substituiert sein kann durch einen Rest ausgewählt aus der Gruppe bestehend aus Hydroxy, Fluor oder CF₃;
- R⁴: C₁-C₃-Alkyl, welches gegebenenfalls substituiert sein kann durch einen Rest ausgewählt aus der Gruppe bestehend aus Hydroxy, Fluor oder CF₃;
- R⁴: ein Phenylrest, der gegebenenfalls substituiert sein kann durch einen oder zwei, bevorzugt einen Rest ausgewählt aus der Gruppe bestehend aus Furyl, Thienyl, Phenyl und Phenyl welches ein-, zwei- oder dreifach durch Methyl, Fluor, Chlor, Brom, Hydroxy, CF₃ oder Methoxy substituiert sein kann, oder
- R⁴: ein Benzylrest, welcher am Phenylring gegebenenfalls substituiert sein kann durch einen, zwei oder drei, bevorzugt einen Rest ausgewählt aus der Gruppe bestehend aus Methyl, Ethyl, Hydroxy, Fluor, Chlor, Brom, CF₃, Methoxy, Furyl, Thienyl und Phenyl, oder
- R⁴: ein Benzylrest, welcher an der Methylenbrücke gegebenenfalls substituiert sein kann durch einen, zwei oder drei, bevorzugt einen Rest ausgewählt aus der Gruppe bestehend aus Methyl, Ethyl, Hydroxy, Fluor, Chlor, Brom, CF₃, Methoxy und Phenyl, oder
- R⁴: ein Rest der Formel 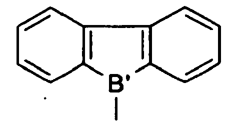 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Von besonderem Interesse sind ferner Verbindungen der allgemeinen Formel **1,** worin
- A: ein zweibindiger Rest ausgewählt aus der Gruppe bestehend aus 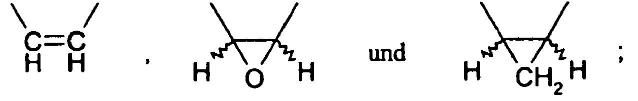
- X⁻: ein einfach negativ geladenes Anion ausgewählt aus der Gruppe bestehend aus Chlorid, Bromid, Methansulfonat und p-Toluolsulfonat, bevorzugt Bromid;
- R¹ und R²: gleich oder verschieden, ein Methyl- oder Ethylrest, der gegebenenfalls durch Cyclopropyl, Hydroxy oder Fluor substituiert sein kann,
oder
R¹ und R² gemeinsam eine C₃-C₄-Alkylen-Brücke;
- R³: Wasserstoff oder C₁-C₃-Alkyl, welches gegebenenfalls substituiert sein kann durch einen Rest ausgewählt aus der Gruppe bestehend aus Hydroxy, Fluor oder CF₃;
- R⁴: C₁-C₃-Alkyl, welches gegebenenfalls substituiert sein kann durch einen Rest ausgewählt aus der Gruppe bestehend aus Hydroxy, Fluor oder CF₃;
- R⁴: ein Phenylrest, der gegebenenfalls substituiert sein kann durch Phenyl, welches gegebenenfalls ein- oder zweifach durch Methyl, Fluor, Hydroxy oder CF₃ substituiert sein kann, oder
- R⁴: ein Benzylrest, welcher am Phenylring gegebenenfalls substituiert sein kann durch einen oder zwei, bevorzugt einen Rest ausgewählt aus der Gruppe bestehend aus Methyl, Ethyl, Hydroxy, Fluor, CF₃, und Phenyl, oder
- R⁴: ein Benzylrest, welcher an der Methylenbrücke gegebenenfalls einfach durch Phenyl substituiert sein kann, oder
- R⁴: ein Rest der Formel 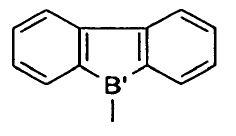 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Von den vorstehend genannten Verbindungen der allgemeinen Formel 1 sind insbesondere diejenigen von besonderer Bedeutung, in denen X⁻ Bromid oder Methansulfonat, besonders bevorzugt Bromid bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Ferner sind erfindungsgemäß diejenigen der vorstehend genannten Verbindungen der Formel **1** besonders bevorzugt, in denen R¹ und R² die gleiche Bedeutung aufweisen und Methyl bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Erfindungsgemäß bedeutsam sind ferner insbesondere diejenigen der vorstehend genannten Verbindungen der Formel **1**, in denen R³ Wasserstoff oder Methyl bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Von besonderer Bedeutung sind ferner all die Verbindungen der allgemeinen Formel **1**, in denen der Rest R⁴ Biphenyl, Benzhydryl, Fluorenyl oder Biphenylmethyl bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Erfindungsgemäß von herausragender Bedeutung sind Verbindungen der allgemeinen Formel 1, worin
- A: ein zweibindiger Rest ausgewählt aus der Gruppe bestehend aus 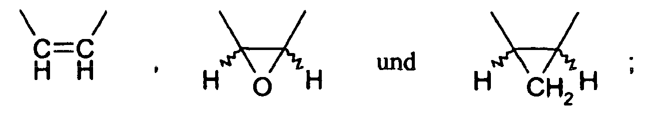
- X⁻: ein einfach negativ geladenes Anion, vorzugsweise ein Anion ausgewählt aus der Gruppe bestehend aus Bromid und Methansulfonat, bevorzugt Bromid;
- R¹ und R²: Methyl;
- R³: Wasserstoff oder Methyl;
- R⁴: Biphenyl, Benzhydryl, Fluorenyl oder Biphenylmethyl,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

Als Alkylgruppen werden, soweit nicht anders angegeben, verzweigte und unverzweigte Alkylgruppen mit 1 bis 5 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 5 Kohlenstoffatomen bezeichnet. Beispielsweise werden genannt: Methylen, Ethylen, n-Propylen oder n-Butylen.

Als Alkyloxygruppen werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor und Brom als bevorzugte Halogene.

Als Alkenylgruppen werden verzweigte und unverzweigte Kohlenwasserstoffgruppen mit 2 bis 5 Kohlenstoffatomen, bevorzugt 2 bis 3 Kohlenstoffatomen bezeichnet, soweit sie mindestens eine Doppelbindung aufweisen. Beispielsweise seien genannt Vinyl, Propenyl, iso-Propenyl, Butenyl, Butadienyl, Pentenyl.

Als Alkinylgruppen werden Kohlenwasserstoffgrupppen mit 2 bis 5 Kohlenstoffatomen bezeichnet, soweit sie mindestens eine Dreifachbindung aufweisen, beispielsweise Ethinyl, Propargyl, Butinyl, Pentinyl.

Der Begriff Aryl steht für ein aromatisches Ringsystem mit 6 bis 10 Kohlenstoffatomen. Bevorzugte Arylreste sind Phenyl oder Naphthyl.

Unter 5-oder 6-gliedriger Heteroarylringen werden im Rahmen der vorliegenden Erfindung aromatische Ringsysteme verstanden, die ein oder zwei Heteroatome ausgewählt aus der Gruppe bestehend aus Sauerstoff, Stickstoff und Schwefel enthalten und wie vorstehend definiert gegebenenfalls substituiert sein können. Beispielsweise werden genannt, Furan, Thiophen, Pyrrol, Pyrazol, Imidazol, Pyridin, Pyrimidin, Triazin, Oxazol, Isoxazol, Thiazol, Isothiazol, Oxadiazol und Pyrazolidin genannt, wobei der Heterocyclus wie in den Definitionen angegeben substituiert sein kann. Besonders bevorzugte 5- oder 6-gliedrige heterocyclische Ringe sind im Rahmen der vorliegenden Erfindung Furan, Thiophen, Pyrrol, Imidazol, Pyridin und Pyrimidin, wobei Furan und Thiophen besondere Bedeutung zukommt.

Der Begriff Arylalkylen steht für aromatische Ringsysteme mit 6 bis 10 Kohlenstoffatomen die über eine Alkylenbrücke mit 1-4-Kohlenstoffatomen verknüpft sind. Als Arylalkylenreste seien beispielsweise genannt Benzyl oder Phenylethyl.

Als 5- oder 6-gliedrige gesättigte oder ungesättigte heterocyclische Ringe, die als Heteroatome Stickstoff, Sauerstoff oder Schwefel enthalten können, werden beispielsweise Furan, Tetrahydrofuran, 2-Methyltetrahydrofuran, 2-Hydroxymethylfuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Oxazol, Isoxazol, Oxazin, Thiazol, Isothiazol, Thiadiazol, Oxadiazol, Pyrazolidin genannt, wobei der Heterocyclus wie in den Definitionen angegeben substituiert sein kann. Besonders bevorzugte 5- oder 6-gliedrige heterocyclische Ringe sind im Rahmen der vorliegenden Erfindung Furan, Thiophen, Pyrrol, Imidazol, Pyridin, Piperidin, Pyrimidin, Morpholin, Oxazol und Oxadiazol, wobei den Ringen Furan, Thiophen, Pyrrol, Imidazol, Pyridin, und Piperidinerfindungsgemäß besondere Bedeutung zukommt.

Als Cycloalkylreste mit 3 - 6 Kohlenstoffatomen werden beispielsweise Cyclopropyl, Cyclobutyl, Cyclopentyl oder Cyclohexyl bezeichnet.

Als Beispiele für 5-, 6- oder 7-gliedrige gesättigte oder ungesättigte heterocyclische Ringe, die durch die Reste R³ und R⁴ gemeinsam mit dem Stickstoff gebildet werden können werden genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen wie in den Definitionen angegeben substituiert sein können. In diesem Zusammenhang werden als besonders bevorzugte Ringe genannt: Pyrrol, Piperidin, Piperazin, N-Methylpiperazin, N-Benzylpiperazin, Morpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen wie in den Definitionen angegeben substituiert sein können.

Unter pharmakologisch verträglichen Säureadditionssalzen werden beispielsweise diejenigen Salze verstanden, die ausgewählt sind aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat.

Die Herstellung der erfindungsgemäßen Verbindungen kann, wie nachstehend erläutert, zum Teil in Analogie zu im Stand der Technik bereits bekannten Vorgehensweisen erfolgen. Schema 1 illustriert die synthetische Vorgehensweise zur Darstellung von Verbindungen der Formel **1** in denen R³ Wasserstoff bedeutet.

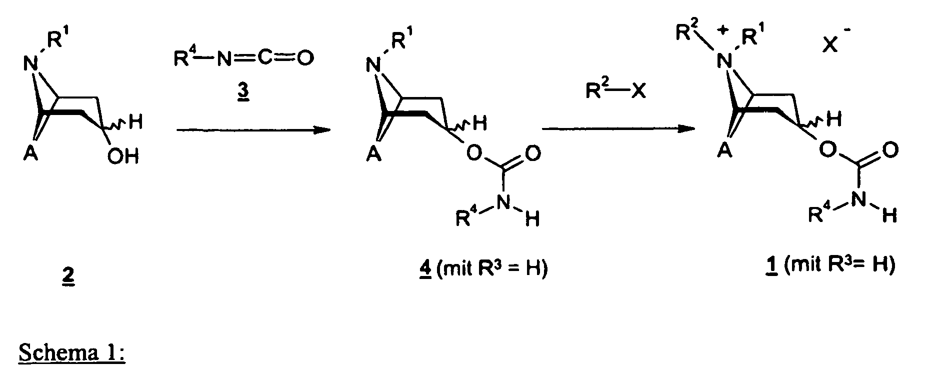

Der Zugang zu diesen Verbindungen gelingt ausgehend von den Alkoholen der Formel **2** durch Umsetzung mit den Isocyanaten der Formel **3.** Die Isocyanate der Formel **3** sind im Stand der Technik bekannt oder können nach im Stand der Technik bekannten Syntheseverfahren erhalten werden. Gegebenenfalls werden die Isocyanate der Formel **3** nach im Stand der Technik bekannten Verfahren in situ generiert und ohne weitere Isloierung in die Reaktion eingesetzt. Die Verbindungen der Formel **2** sind ebenfalls im Stand der Technik bekannt. Die Umsetzung der Verbindungen **2** mit den Isocyanaten **3** wird vorzugsweise in einem aprotischen organischen Lösemittel, besonders bevorzugt in einem aprotischen polaren Lösemittel beispielsweise ausgewählt aus der Gruppe bestehend aus Acetonitril, Tetrahydrofuran und Dioxan, bevorzugt Acetonitril bei Temperaturen von 0-100°C, bevorzugt bei etwa 10-50°C besonders bevorzugt bei Raumtemperatur (etwa 23°C) durchgeführt. Die Aufarbeitung der Reaktionsmischung und Reinigung des Zwischenprodukts **4** erfolgt nach üblichen Vorgehensweisen. Verbindungen der Formel **4**, in denen R³ Wasserstoff bedeutet, sind teilweise im Stand der Technik bekannt. Diesbezüglich sei auf die Veröffentlichungen WO 97/34892, Aberle et al. (Tetrahedron Letters 42 (2001) 1975-1977) sowie Polonovski et al. (Bull. Soc. Chim. Fr. <4> 43, 1928, 596) verwiesen.

Die Verbindungen der Formel **1**, in denen R³ nicht Wasserstoff bedeutet sind gemäß Schema 2 zugänglich.

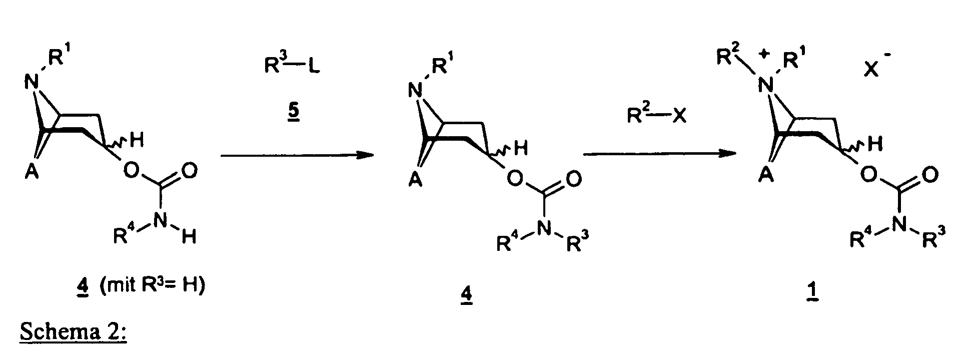

Hierzu wird eine Verbindung der Formel **4,** in der R³ Wasserstoff bedeutet, in einem geeigneten organischen Lösemittel in Gegenwart einer starken Base mit der Verbindung **5** umgesetzt. In der Verbindung **5** kann R³ die oben genannten Bedeutungen haben. L steht für eine Abgangsgruppe, bevorzugt für eine Abgangsgruppe ausgewählt aus Chlor, Brom, Iod, Mesylat, Triflat, Benzolsulfonat oder Tosylat. Erfindungsgemäß kommen als Basen vorzugsweise Alkalihydride in Betracht. Die Hydride des Natriums, Lithiums und Kaliums sind bevorzugt. Geeignete Lösungsmittel sind vorzugsweise ausgewählt aus Dimethylformamid, Dimethylacetamid, Methylenchlorid und Tetrahydrofuran. Das Reaktionsgemisch wird 0,5 bis 4 Tage, bevorzugt 1 bis 2 Tage bei Raumtemperatur, gegebenenfalls auch bei erhöhter Temperatur, durchgeführt. Die Aufarbeitung der Reaktionsmischung und Reinigung des Produkts **4** erfolgt nach üblichen Vorgehensweisen.

Die gemäß Schema 1 oder Schema 2 erhaltenen Verbindungen der Formel **4** lassen sich durch Umsetzung mit den Verbindungen R²-X, in denen R² und X die vorstehend genannten Bedeutungen haben können, in die Zielverbindungen der Formel **1** überführen. Auch die Durchführung dieses Syntheseschritts kann in Analogie zu den in der WO 92/16528 offenbarten Synthesebeispielen erfolgen. In dem Fall in dem R¹ und R² gemeinsam eine Alkylenbrücke bilden, ist, wie für den Fachmann ersichtlich, die Zugabe des Reagenzes R²-X nicht erforderlich. In diesem Fall weisen die Verbindungen der Formel 4 einen geeignet substituierten Rest R¹ (-C₃-C₅-Alkylen-X) entsprechend der vorstehend genannten Definitionen auf und die Darstellung der Verbindungen der Formel **1** erfolgt durch intramolekulare Quartemierung des Amins.

Wie in Schema 1 ersichtlich, kommt den Zwischenprodukten der allgemeinen Formel **4** eine zentrale Bedeutung zu.

Dementsprechend zielt ein weiterer Aspekt der vorliegenden Erfindung auf die Intermediate der Formel **4** 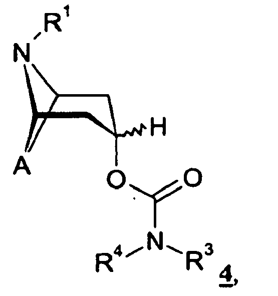 worin
- A: ein zweibindiger Rest ausgewählt aus der Gruppe bestehend aus 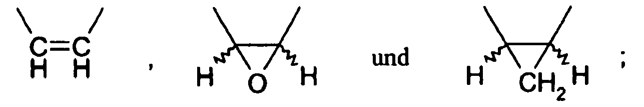
- R¹: , ein Methyl- oder Ethylrest, der gegebenenfalls durch Cyclopropyl, Hydroxy oder Fluor substituiert sein kann, oder
- R¹: C₃-C₄-Alkylen-X, wobei X Chlorid, Bromid, Methansulfonat und p-Toluolsulfonat;
- R³: Wasserstoff oder C₁-C₃-Alkyl, welches gegebenenfalls substituiert sein kann durch einen Rest ausgewählt aus der Gruppe bestehend aus Hydroxy, Fluor oder CF₃;
- R⁴: C₁-C₃-Alkyl, welches gegebenenfalls substituiert sein kann durch einen Rest ausgewählt aus der Gruppe bestehend aus Hydroxy, Fluor oder CF₃, oder
- R⁴: ein Phenylrest, der substituiert ist durch Phenyl, welches gegebenenfalls ein- oder zweifach durch Methyl, Fluor, Hydroxy oder CF₃ substituiert sein kann, oder
- R⁴: ein Benzylrest, welcher am Phenylring gegebenenfalls substituiert sein kann durch einen oder zwei, bevorzugt einen Rest ausgewählt aus der Gruppe bestehend aus Methyl, Ethyl, Hydroxy, Fluor, CF₃, und Phenyl, oder
- R⁴: ein Benzylrest, welcher an der Methylenbrücke gegebenenfalls einfach durch Phenyl substituiert sein kann, oder
- R⁴: ein Rest der Formel 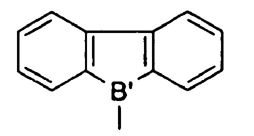 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer Säureadditionssalze, Solvate und Hydrate,

Erfindungsgemäß von herausragender Bedeutung sind Intermediate der allgemeinen Formel **4,** worin
- A: ein zweibindiger Rest ausgewählt aus der Gruppe bestehend aus 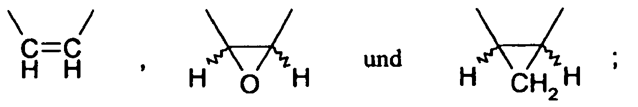
- R¹: Methyl;
- R³: Wasserstoff oder Methyl;
- R⁴: Biphenyl, Benzhydryl, Fluorenyl oder Biphenylmethyl,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer Säureadditionssalze, Solvate und Hydrate.

Unter Säureadditionssalzen werden dabei Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotarnat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Ferner betrifft die vorliegende Erfindung die Verwendung der vorstehend genannten Intermediate der Formel **4** zur Herstellung der Verbindungen der Formel **1.**

Die nachstehend beschriebenen Synthesebeispiele dienen der weitergehenden Illustration der vorliegenden Erfindung.

### Beispiel 1: Biphen-2-ylcarbaminsäuretropenolester-Methobromid

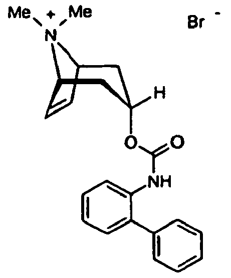

### 1.1.: Biphen-2-ylcarbaminsäuretropenolester

5,0 g (0,026 mol) 2-Biphenylisocyanat werden in 10 ml Acetonitril vorgelegt, eine Lösung aus 3,619 g (0,026 mol) Tropenol in 5 ml Acetonitril zugetropft, anschließend 24 Stunden bei Raumtemperatur gerührt. Die Lösung wird eingedampft und der Rückstand zwischen Dichlormethan und Wasser verteilt. Die organische Phase wird über Natriumsulfat getrocknet und zur Trockene eingedampft.
Ausbeute: 7,4 g gelborangenes Öl (= 85% d. Th.).

### 1.2.: Biphen-2-ylcarbaminsäuretropenolester-Methobromid

1,00 g (0,003 mol) Biphenyl-2-ylcarbaminsäuretropenolester und 1,709 g (0,009 mol) 50%ige Methylbromidlösung in Acetonitril werden in 70 ml Acetonitril vorgelegt und 3 Tage bei Raumtemperatur verschlossen und abgedunkelt stehen lassen. Es wird zum Rückstand eingedampft. Die Reinigung erfolgt durch Umkristallisation erst aus Ethanol / Diethylether. Ausbeute: 0,38 g weiße Kristalle (= 29% d. Th.); Smp.: 244°-245° C.

### Beispiel 2: Biphen-2-ylcarbaminsäurescopinester-Methobromid

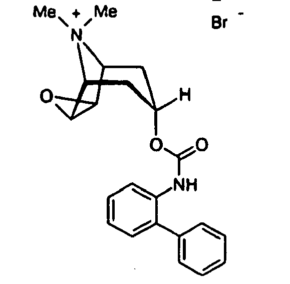

### 2.1.: Biphen-2-ylcarbaminsäurescopinester-Hydrochlorid

2,5 g (0,013 mol) 2-Biphenyl-isocyanat werden in 7 ml Acetonitril vorgelegt, eine Lösung aus 0,99 g (0,013 mol) Scopin in 7 ml Acetonitril bei Raumtemperatur zugetropft. Nach 3 Stunden Reaktionszeit wird die Lösung eingedampft und der Rückstand zwischen Dichlormethan und Wasser verteilt. Die organische Phase wird über Natriumsulfat getrocknet und zur Trockene eingedampft. Der Rückstand wird in Dichlormethan gelöst, durch Zugabe von etherischer HCl gefällt, anschließend in Wasser gelöst und mit Diethylether gewaschen. Die wässrige Phase wird mittels 10%iger Natriumcarbonatlösung basisch gestellt und mit Dichlormethan extrahiert. Anschließend wird das Hydrochlorid gefällt. Ausbeute: 1,57 g (= 31% d. Th.); Smp.: 154°-155° C.

### 2.2.: Biphen-2-ylcarbaminsäurescopinester-Methobromid

Aus 1,56 g (0,004 mol) Biphen-2-ylcarbaminsäurescopinester-Hydrochlorid wird die freie Base durch Versetzen mit 10%iger Natriumcarbonat-Lösung und anschließende Extraktion mit Methylenchlorid hergestellt. Diese wird nach Trocknung über Magnesiumsulfat zur Trockene eingedampft, in 40 ml Acetonitril und 20 ml Dichlormethan gelöst, mit 3,5 g (0,012 mol) ca. 50%iger Methylbromidlösung in Acetonitril versetzt und 6 Tage bei Raumtemperatur verschlossen und abgedunkelt stehen lassen. Die Lösung wird zur Trockene eingedampft. Die Reinigung erfolgt durch Umkristallisation aus Ethanol. Ausbeute: 1,33 g (= 75% d. Th.); Smp.: 216°-217° C.

### Beispiel 3: Biphen-2-yl-methyl-carbaminsäuretropenolester-Methobromid

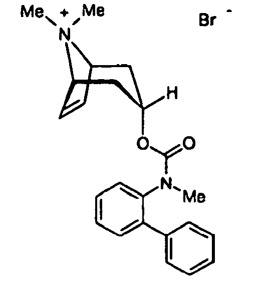

### 3.1.: Biphen-2-yl-methyl-carbaminsäurescopinester-Hydrochlorid

2,5 g (0,007 mol) Biphenyl-2-ylcarbaminsäuretropenolester werden bei Raumtemperatur in 40 ml Tetrahydrofuran gelöst, mit 0,45 g (0,011 mol) Natriumhydrid versetzt und 0,5 Stunden bei Raumtemperatur gerührt. Anschließend werden 1,06 g (0,007 mol) Methyljodid zugetropft und die Reaktionsmischung 24 Stunden bei Raumtemperatur gerührt. Die erhaltene Suspension wird zur Trockene eingedampft und zwischen Dichlormethan und Wasser verteilt. Die organische Phase wird mit essigsaurem Wasser (2 Tropfen Eisessig auf 100 ml Wasser) gewaschen, über Magnesiumsulfat getrocknet und zur Trockene eingedampft. Der Rückstand wird in Dichlormethan gelöst, mittels etherischer HCl gefällt und aus Isopropanol / Diethylether umkristallisiert.
Ausbeute: 0,23 g (= 9% d. Th.); Smp.: 192°-194° C.

### 3.2.: Biphen-2-yl-methyl-carbaminsäurescopinester-Methobromid

Aus 0,2 g (0,001 mol) Biphenyl-2-yl-methyl-carbaminsäurescopinester-Hydrochlorid wird die freie Base durch Versetzen mit 10%iger Natriumcarbonat-Lösung und anschließende Extraktion mit Methylenchlorid hergestellt. Der Rückstand wird in 50 ml Acetonitril und 30 ml Dichlormethan gelöst und mit 0,596 g (0,003 mol) ca. 50%iger Methylbromidlösung in Acetonitril versetzt. Die Lösung wird 4 Tage verschlossen und abgedunkelt bei Raumtemperatur stehen lassen, dann das Lösemittel unter vermindertem Druck abdestilliert. Die Reinigung erfolgt durch Umkristallisation aus Acetonitril.
Ausbeute: 0,06 g (= 14% d. Th.); Smp.: 228°-229° C.

### Beispiel 4: Benzhydryl-carbaminsäuretropenolester-Methobromid

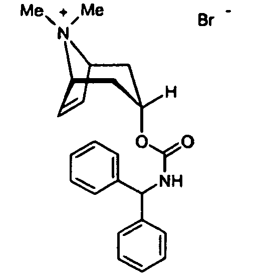

### 4.1.: Benzhydryl-carbaminsäuretropenolester

2,5 g (0,012 mol) Diphenylmethylisocyanat werden in 7 ml Acetonitril vorgelegt, eine Lösung aus 1,67 g (0,012 mol) Tropenol in 5 ml Acetonitril zugetropft. Die Lösung wird 24 Stunden bei Raumtemperatur gerührt, nach 10 Minuten bildet sich ein weißer Niederschlag. Nach Beenden der Reaktionszeit wird die Suspension abgekühlt und der Niederschlag abgesaugt. Ausbeute: 2,28 g (= 55% d. Th.); Smp.: 140°-142° C.

### 4.2.: Benzhydryl-carbaminsäuretropenolester-Methobromid

1,2 g (0,003 mol) Benzhydryl-carbaminsäuretropenolester werden in 40 ml Acetonitril und 30 ml Dichlormethan gelöst, 1,789 g (0,009 mol) ca. 50%ige Methylbromidlösung in Acetonitril zugegeben und 4 Tage verschlossen bei Raumtemperatur stehen lassen. Die Lösung wird eingedampft und der Rückstand aus Acetonitril umkristallisiert.
Ausbeute: 0,76 g (= 57% d. Th.); Smp.: 243°-244° C.

### Beispiel 5: 9H-Fluoren-9-yl-carbaminsäuretropenolester-Methobromid

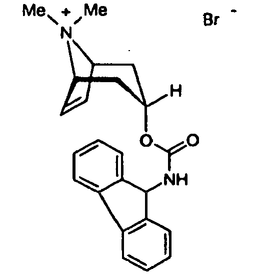

### 5.1.: 9H-Fluoren-9-yl -carbaminsäuretropenolester

1,95 g (0,009 mol) 9-Fluoren-9-isocyanat werden in 20 ml Acetonitril suspendiert, eine Lösung aus1,253 g (0,009 mol) Tropenol in 4 ml Acetonitril bei Raumtemperatur zugetropft, dann 24 Stunden bei der Temperatur gerührt. Das Reaktionsgemisch wird im Vakuum eingedampft und zwischen Dichlormethan und Wasser verteilt. Die organische Phase wird über Magnesiumsulfat getrocknet und zur Trockene eingedampft. Der Rückstand wird in Dichlormethan gelöst, durch Zugabe von etherischer HCl gefällt, anschließend in Wasser gelöst und mit Diethylether gewaschen. Der erhaltene Feststoff wird in Wasser gelöst, mit 10%iger Natriumcarbonatlösung versetzt, mit Dichlormethan extrahiert, über Magnesiumsulfat getrocknet, zur Trockene eingedampft und aus Acetonitril umkristallisiert. Ausbeute: 0,32 g (= 10% d. Th.); Smp.: 143°-144° C.

### 5.2.: 9H-Fluoren-9-yl -carbaminsäuretropenolester-Methobromid

0,32 g (0,001 mol) 9H-Fluoren-9-yl -carbaminsäuretropenolester werden in 30 ml Acetonitril und 50 ml Dichlormethan gelöst, 0,596 g (0,003 mol) ca. 50%ige Methylbromidlösung in Acetonitril zugegeben. Die Lösung wird 4 Tage bei Raumtemperatur verschlossen stehen lassen, dann zum Rückstand eingedampft und aus Acetonitril umkristallisiert. Ausbeute: 0,16 g (= 36% d. Th.); Smp: 226°-227° C.

### Beispiel 6: Biphen-2-ylmethyl-carbaminsäuretropenolester-Methobromid

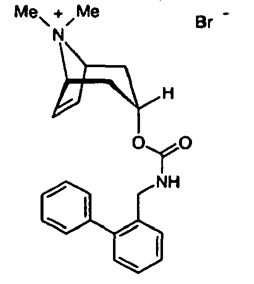

### 6.1.: Biphen-2-ylmethyl-carbaminsäuretropenolester

3,67 g (0,02 mol) 2-Phenylbenzylamin werden in 250 ml gesättigter Natriumbicarbonatlösung und 250 ml Dichlormethan vorgelegt. Bei 0° C werden 52,9 ml (0,10 mol) 20%ige Phosgen-Toluollösung unter die Dichlormethanoberfläche zugegeben. Es wird 30 Minuten heftig gerührt. Die zwei Phasen werden getrennt, die organische Phase über Natriumsulfat getrocknet und zur Trockene eingedampft.
Der Rückstand wird in 20 ml Acetonitril gelöst und 4,18 g (0,03 mol) Tropenol in 10 ml Acetonitril zugetropft, wobei ein Niederschlag ausfällt. Es wird 12 Stunden bei Raumtemperatur gerührt. Der Niederschlag wird abgesaugt und die Mutterlauge zur Trockene eingedampft. Der Rückstand wird in Dichlormethan gelöst und mit Wasser, essigsaurem Wasser (2 Tropfen Eisessig auf 100 ml Wasser) und 10 %iger Natriumcarbonatlösung gewaschen. Die organische Phase wird getrocknet und zur Trockene eingedampft. Ausbeute: 2,85 g (= 41% d. Th.).

### 6.2.: Biphen-2-ylmethyl-carbaminsäuretropenolester-Methobromid

2,8 g (0,008 mol) Biphenyl-2-ylmethyl-carbaminsäuretropenolester werden in 10 ml Acetonitril gelöst und mit 2,27 g (0,024 mol) 50%iger Methylbromidlösung versetzt. Die Lösung wird 4 Tage verschlossen und abgedunkelt bei Raumtemperatur stehen lassen. Das Lösungsmittel wird eingedampft und der schaumige Rückstand zur vollständigen Kristallisation mit Aceton verrieben. Ausbeute: 2,78 g (= 78% d. Th.); Schmp.180°C-181°C (Zers.).

### Beispiel 7: Biphen-2-ylmethyl-carbaminsäurescopinester-Methobromid

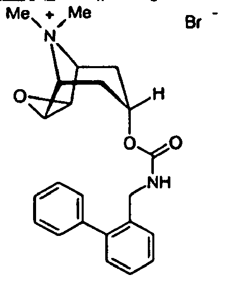

### 7.1.: Biphenyl-2-ylmethyl-carbaminsäurescopinester

3,67 g (0,02 mol) 2-Phenylbenzylamin werden in 250 ml gesättigter Natriumbicarbonatlösung und 250 ml Dichlormethan vorgelegt. Bei 0° C werden 52,9 ml (0,10 mol) 20%ige Phosgen-Toluollösung unter die Dichlormethanoberfläche zugegeben. Es wird 30 Minuten heftig gerührt. Die zwei Phasen werden getrennt, die organische Phase über Natriumsulfat getrocknet und zur Trockene eingedampft.
Der Rückstand wird in 20 ml Acetonitril gelöst und 4,66 g (0,03 mol) Scopin in 10 ml Acetonitril zugetropft, wobei ein Niederschlag ausfällt. Es wird 12 Stunden bei Raumtemperatur gerührt. Der Niederschlag wird abgesaugt und die Mutterlauge zur Trockene eingedampft. Der Rückstand wird in Dichlormethan gelöst, mit Wasser gewaschen und anschließend mit verdünnter Salzsäure (ca. 0,05 mol/L) extrahiert. Die saure Wasserphase wird mit Natriumcarbonat basisch gestellt und mit Dichlormethan extrahiert. Die daraus resultierende organische Phase wird über Natriumsulfat getrocknet und zur Trockene eingedampft. Ausbeute: 2,85 g (= 33% d. Th.)

### 7.2.: Biphen-2-ylmethyl-carbaminsäurescopinester-Methobromid

1,2 g (0,003 mol) Biphenyl-2-ylmethyl-carbaminsäurescopinester werden in 5 ml Acetonitril gelöst und mit 1,709 g (0,009 mol) 50%iger Methylbromidlösung versetzt. Die Lösung wird 4 Tage verschlossen und abgedunkelt bei Raumtemperatur stehen lassen. Das Lösungsmittel wird eingedampft und der schaumige Rückstand zur vollständigen Kristallisation mit Aceton verrieben. Ausbeute: 1,04 g (= 76% d. Th.); Schmp.175°C-176°C.

### Beispiel 8: Biphen-2-ylcarbaminsäure-9-methyl-9-aza-tricyclo[3.3.1.0*2.4*]non-7-yl ester-Methobromid

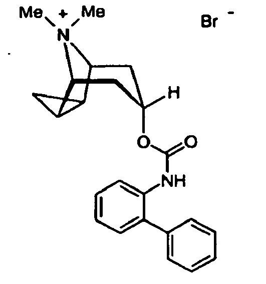

### 8.1.: 9-Methyl-9-aza-tricyclo[3.3.1.0*2,4*]nonan-7-ol

35 ml (0,349 mol) 40%aq. KOH wurde mit 100 ml Diethylether überschichtet und im Eisbad gekühlt, 23,64 g (0,101 mol) N-Methyl-N-Nitrosoharnstoff portionsweise zugegeben, 10 min. nachgerührt. Anschließend wurde die Etherphase abdekantiert, nochmals mit Diethylether versetzt, umgeschwenkt und abdekantiert. Die vereinten org. Phasen wurden über festem KOH unter Eisbadkühlung getrocknet, die erhaltene Lösung weiterverwendet.
Zu einer Lösung aus 4,01 g (0,028 mol) Tropenol in 25 ml Diethylether und 5 ml Methanol wurden unter Eisbadkühlung 25 ml der oben hergestellten Diazomethanlösung zugegeben, danach 53,4 mg (0,000139 mol) Bis(benzonitril)dichloro-palladium(II). Es erfolgte weitere Zugabe von 8 ml der Diazomethanlösung, 2 malige Zugabe von jeweils 10 ml Diazomethanlösung nach je 30 min, dann wurden noch 30 min. nachgerührt. Das Lösungsmittel wurde eingedampft, mit heißem Hexan extrahiert, heiß filtriert, zur Trockene eingedampft. Ausbeute: 4,25 g leicht gelbliche Kristalle (= 96% d. Th.).

### 8.2.: Biphen-2-ylcarbarninsäure-9-methyl-9-aza-tricyclo[3.3.1.0*2.4*]non-7-yl ester-Hydrochlorid

Zu einer Lösung von 2,5 g Biphenyl-2-isocyanat in Acetonitril wurden 2,15 g 9-Methyl-9-aza-tricyclo[3.3.1.0*2,4*]nonan-7-ol in Acetonitril getropft. Die Lösung wurde über Nacht bei Raumtemperatur gerührt, mit 200 ml Dichlormethan verdünnt und mit Wasser und essigsaurem Wasser (2 Tropfen Eisessig auf 100 ml Wasser) gewaschen. Die organische Phase wurde über Magnesiumsulfat getrocknet, filtriert und einrotiert. Das erhaltene, gelbe Öl wurde in Dichlormetham gelöst, durch Zugabe von etherischer HCl in das Hydrochlorid überführt und aus Acetonitril/Ether umkristallisiert. Ausbeute: 0,35g weiße Kristalle (= 7% der Th.).

### 8.3.: Biphenyl-2-ylcarbaminsäure-9-methyl-9-aza-tricyclo[3.3.1.0*2.4*]non-7-yl ester-Methobromid

0,35 g Biphenyl-2-ylcarbaminsäure-9-methyl-9-aza-tricyclo[3.3.1.0*2,4*]non-7-yl ester-Hydrochlorid wurden in Wasser gelöst, mit 10%iger Natriumcarbonat-Lösung basisch gestellt und mit Dichlormethan extrahiert. Die organische Phase wurde über Magnesiumsulfat getrocknet, filtriert und zur Trockene eingedampft. Der Rückstand wurde in 10 ml Acetonitril gelöst, und nach Zugabe von 1,2 g 50%iger Methylbromidlösung in Acetonitril im verschlossenen Druckgefäß 30 Tage bei 75° C gerührt. Die Lösung wurde zur Trockene eingedampft und der Rückstand aus Acetonitril umkristallisiert.
Ausbeute: 0,16g weiße Kristalle (= 39% der Th.); Schmelzpunkt: 142-144°C.

Wie gefunden wurde, stellen die erfindungsgemäßen Verbindungen der Formel 1 Antagonisten des M3- Rezeptors (Muskarinischer Rezeptorsubtyp 3) dar. Die erfindungsgemäßen Verbindungen weisen bezüglich der Affinität zum M3-Rezeptor Ki-Werte von kleiner 1000nM auf. Diese Werte wurde entsprechend der nachfolgend beschriebenen Vorgehensweise bestimmt.

### Chemikalien

3H-NMS wurde von der Firma Amersham, Braunschweig, mit einer spezifischen Radioaktivität von 3071 GBq/mmol (83 Ci/mmol) bezogen. Alle weiteren Reagentien wurden von Serva, Heidelberg und von Merck, Darmstadt erhalten.

### Zellmembranen:

Wir setzten Zellmembranen von CHO Zellen (Chinese hamster ovary) ein, die mit den entsprechenden Genen der humanen muskarinischen Rezeptorsubtypen hm1 bis hm5 transfiziert waren (BONNER). Die Zellmembranen des gewünschten Subtypes wurden aufgetaut, resuspendiert per Hand mit einem Glas-Homogenisator und mit HEPES-Puffer auf eine Endkonzentration von 2-30 mg Protein/ml verdünnt.

### Rezeptorbindungsstudien:

Der Bindungsassay wurde in einem Endvolumen von 200µl ausgeführt und setzte sich zusammen aus 50 µl unmarkierter Substanz in verschiedenen Konzentrationen, 50 µl Radioligand (3H-N-Methylscopolamin 2 nmol/L (3H-NMS)), 100 µl Membranpräparation in TRIS-Puffer (50 mmol/L TRIS, 10 mmol/L MgCl2, 100 mmol/L NaCl, mit 1 mol/L NaOH auf pH 7.4 eingestellt).
Die unspezifische Bindung ermittelten wir durch Verdrängung mit 10 µmol/L Atropin. Die Inkubation erfolgte bei Raumtemperatur eine Stunde in 96-well Mikrotiterplatten (Beckman, Polystyrol, Nr. 267001) als Doppelbestimmung. Die Inkubation endete durch Filtration mittels Skatron Zellernter (Typ IH 110) über Whatman GF/C Filter. Die Filter wurden mit 3 ml eisgekühltem TRIS Puffer gewaschen. Anschließend wurden die Filter in Scintillationsvials (Zinsser 5 ml) überführt, mit 4 ml Scintillationsflüssigkeit versetzt (Zinsser: Quickszint 2000 bzw. Beckmann Ultima Gold) und 1 h über Kopf geschüttelt.

### Bestimmung der Radioaktivität:

Die Radioaktivität der Filter wurde am Szintillationscounter (Beckmann bzw. LKB) gemessen.

### Auswertung:

Die Ki-Werte berechneten wir mit impliziten Gleichungen, die direkt vom Massenwirkungsgesetz abgeleitet wurden, mit dem Modell für die 1 Rezeptor 2 Liganden Reaktion (SysFit - Software, SCHITTKOWSKI).

### Literatur:

BONNER TI, New subtypes of muscarinic acetylcholine receptors Trends Pharmacol. Sci. 10, Suppl.: 11-15 (1989); SCHITTKOWSKI K Parameter estimation in systems of nonlinear equations Numer Math. 68: 129-142 (1994).

Die erfindungsgemäßen Verbindungen der Formel **1** zeichnen sich durch vielfältige Anwendungsmöglichkeiten auf therapeutischem Gebiet aus.
Hervorzuheben sind erfindungsgemäß solche Anwendungsmöglichkeiten, für welche die erfindungsgemäßen Verbindungen der Formel **1** aufgrund ihrer pharmazeutischen Wirksamkeit als Anticholinergikum bevorzugt zur Anwendung gelangen können.
Dies sind beispielsweise die Therapie von Asthma oder COPD (chronic obstructive pulmonary disease = chronisch obstruktive Lungenerkrankung). Die Verbindungen der allgemeinen Formel **1** können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei Menstruationsbeschwerden zum Einsatz gelangen.
Von den vorstehend beispielhaft aufgeführten Indikationsgebieten, kommt der Therapie von Asthma und COPD mittels der erfindungsgemäßen Verbindungen der Formel **1** eine besondere Bedeutung zu.

Die Verbindungen der allgemeinen Formel **1** können allein oder in Kombination mit anderen erfindungsgemäßen Wirkstoffen der Formel **1** zur Anwendung gelangen. Gegebenenfalls können die Verbindungen der allgemeinen Formel **1** auch in Kombination mit weiteren pharmakologisch aktiven Wirkstoffen eingesetzt werden.
Es handelt sich hierbei insbesondere um Betamimetica, Antiallergika, PAF-Antagonisten, PDE IV-Inhibitoren, Leukotrien-Antagonisten, p38 Kinase-Inhibitoren, EGFR-Kinase-Hemmer und Corticosteroiden, sowie Wirkstoffkombinationen davon.

Als Beispiel für Betamimetika, die erfindungsgemäß mit den Verbindungen der Formel **1** als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze, ihrer Solvate und/oder ihrer Hydrate. Besonders bevorzugt gelangen als Betamimetika solche Wirkstoffe in Kombination mit den erfindungsgemäßen Verbindungen der Formel **1** zur Anwendung, die ausgewählt sind aus der Gruppe bestehend aus Fenoterol, Formoterol, Salmeterol, 1-[3-(4-Methoxybenzylamino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.
Erfindungsgemäß bevorzugt sind die Säureadditionssalze der Betamimetika beispielsweise ausgewählt aus der Gruppe bestehend aus Hydrochlorid, Hydrobromid, Sulfat, Phosphat, Fumarat, Methansulfonat und Xinafoat. Besonders bevorzugt sind die Salze im Falle des Salmeterols ausgewählt aus Hydrochlorid, Sulfat und Xinafoat, von denen das Xinafoate besonders bevorzugt ist. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylline, Roflumilast, Ariflo, Bay-198004, CP-325,366, BY343, D-4396 (Sch-351591), V-11294A und AWD-12-281. Bevorzugte PDE-IV-Inhibitoren sind ausgewählt aus der Gruppe bestehend aus Enprofylline, Roflumilast, Ariflo und AWD-12-281, wobei AWD-12-281 als Kombinationspartner mit der erfindungsgemäßen Verbindung der Formel **1** besonders bevorzugt ist. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-IV-Inhibitoren gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Im Rahmen der vorliegenden Erfindung werden unter Dopamin-Agonisten, die gegebenenfalls in Kombination mit den Verbindungen der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel **1** zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt
4-(2-Chlorphenyl)-9-methyl-2-[3(4-morpholinyl)-3-propanon-1-yl]-6H-thieno-[3,2-f] [1,2,4]triazolo[4,3-a][1,4]diazepin,
6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin.

Als Beispiel für EGFR-Kinase-Hemmer, die mit den erfindungsgemäßen Verbindungen der Formel **1** als Kombination zum Einsatz kommen können, seien als besonders bevorzugt genannt 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, 4-[(3-Chlor-4-fluor-phenyl)amino]-7-(2-{4-[(S)-(2-oxo-tetrahydrofiwan-5-yl)carbonyl]-piperazin-1-yl}-ethoxy)-6-[(vinylcarbonyl)amino]-chinazolin, 4-[(3-Chlor-4-fluorphenyl)amino]-7-[2-((S)-6-methyl-2-oxo-morpholin-4-yl)-ethoxy]-6-[(vinylcarbonyl)amino]-chinazolin, 4-[(3-Chlor-4-fluorphenyl)amino]-6-[(4- {N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopropylrnethoxy-chinazolin und 4-[(3-Chlor-4-fluorphenyl)amino]-6-[3-(morpholin-4-yl)-propyloxy]-7-methoxychinazolin. Eine Bezugnahme auf die vorstehend genannten EGFR-Kinase-Hemmer schließt im Rahmen der vorliegenden Erfindung eine Bezugnahme auf deren gegebenenfalls existierende pharmakologisch verträgliche Säureadditionssalze ein. Unter den physiologischbzw. pharmakologisch verträglichen Säureadditionssalzen, die von den EGFR-Kinase-Hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der EGFR-Kinase-Hemmer ausgewählt aus den Salzen der Essigsäure, Salzsäure, Bromwasserstoffsäure, Schwefelsäure, Phosphorsäure und Methansulfonsäure.

Als Beispiel für p38 Kinase-Hemmer, die mit den erfindungsgemäßen Verbindungen der Formel **1** als Kombination zum Einsatz kommen können, seien als besonders bevorzugt genannt 1-[5-*tert*-Butyl-2-*p*-tolyl-2H-pyrazol-3-yl]-3-[4-(2-morpholin-4-yl-ethoxy)naphthalin-1-yl]-harnstoff; 1-[5-*tert*-Butyl-2-*p*-tolyl-2H-pyrazol-3-yl]-3-[4-(2-(1-oxothiomorpholin-4-yl)ethoxy)naphthalin-1-yl]-harnstoff; 1-[5-tert-butyl-2-(2-methylpyridin-5-yl)-2H-pyrazol-3-yl]-3-[4-(2-pyridin-4-yl-ethoxy)naphthalin-1-yl]-harnstoff; 1-[5-tert-butyl-2-(2-methoxypyridin-5-yl)-2H-pyrazol-3-yl]-3-[4-(2-morpholin-4-yl-ethoxy)naphthalin-1-yl]-harnstoff oder 1-[5-tert-butyl-2-methyl-2H-pyrazol-3-yl]-3-[4-(2-morpholin-4-yl-ethoxy)naphthalen-1-yl]-harnstoff. Eine Bezugnahme auf die vorstehend genannten p38-Kinase-Hemmer schließt im Rahmen der vorliegenden Erfindung eine Bezugnahme auf deren gegebenenfalls existierende pharmakologisch verträgliche Säureadditionssalze ein. Unter den physiologisch bzw. pharmakologisch verträglichen Säureadditionssalzen, die von den p38-Kinase-hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind.

Werden die Verbindungen der Formel **1** in Kombination mit anderen Wirkstoffen eingesetzt, ist von den vorstehend genannten Verbindungsklassen die Kombination mit Steroiden, PDE IV-inhibitoren oder Betamimetika besonders bevorzugt. Der Kombination mit Betamimetika, insbesondere mit langwirksarnen Betamimetika kommt dabei eine besondere Bedeutung zu. Als besonders bevorzugt ist die Kombination der erfindungsgemäßen Verbindungen der Formel **1** mit Salmeterol oder Formoterol anzusehen.

Geeignete Anwendungsformen zur Applikation der Verbindungen der Formel **1** sind beispielsweise Tabletten, Kapseln, Zäpfchen, Lösungen etc.
Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdispeise Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.
Sind die Wirkstoffe 1 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt.
Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDls = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff **1** | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff **1** | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff **1** | 0,005 |
| | Sorbitantrioleat | 0,1 |
| | Monofluortrichlormethan und | |
| | Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff **1** | 333.3 mg |
| | Formoterolfumarat | 333.3 mg |
| | Benzalkoniumchlorid | 10.0 mg |
| | EDTA | 50.0 mg |
| | HCl(ln) | ad pH 3.4 |

Diese Lösung kann in üblicher Art und Weise hergestellt werden.

| F) | Inhalationpulver | |
|---|---|---|
| | Wirkstoff **1** | 8 µg |
| | Formoterolfumarat | 6 µg |
| | Lactose Monohydrat | ad 25 mg |

Die Herstellung des Inhaltionspulvers erfolgt in üblicher Art und Weise durch Mischen der einzelnen Bestandteile.

| G) | Inhalationpulver | |
|---|---|---|
| | Wirkstoff **1** | 9 µg |
| | Lactose Monohydrat | ad 5 mg |

Die Herstellung des Inhaltionspulvers erfolgt in üblicher Art und Weise durch Mischen der einzelnen Bestandteile.

## Patentansprüche

1. Verbindungen der allgemeinen Formel **1** 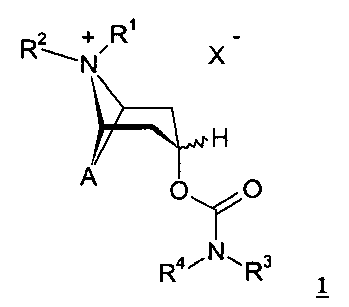 worin
A ein zweibindiger Rest ausgewählt aus der Gruppe bestehend aus 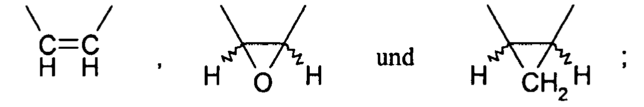
X⁻ ein einfach negativ geladenes Anion;
R¹ und R² gleich oder verschieden, C₁-C₅-Alkyl, welches gegebenenfalls durch einen Rest ausgewählt aus der Gruppe bestehend aus -C₃-C₆-Cycloalkyl, Hydroxy oder Halogen substituiert sein kann,
oder
R¹ und R² gemeinsam eine C₃-C₅-Alkylen-Brücke;
R³ und R⁴ gleich oder verschieden,
Wasserstoff, oder
C₁-C₅-Alkyl, welches gegebenenfalls ein- oder mehrfach substituiert sein kann durch einen oder mehrere Reste ausgewählt aus der Gruppe bestehend aus Hydroxy, Halogen, CF₃ und
-OC₁-C₄-Alkyl, oder
eine C₂-C₅-Alkenyl- oder C₂-C₅-Alkinyl-Gruppe, welche gegebenenfalls ein- oder mehrfach substituiert sein kann durch einen oder mehrere Reste ausgewählt aus der Gruppe bestehend aus Hydroxy, Halogen, CF₃, -OC₁-C₄-Alkyl, Phenyl und Phenyl, welches ein- oder mehrfach durch Methyl, Halogen, Hydroxy, CF₃ oder Methoxy substituiert sein kann, oder
C₆-C₁₀-Aryl, welches gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl, welches ein- oder mehrfach durch Methyl, Halogen, Hydroxy, CF₃ oder Methoxy substituiert sein kann, oder
C₆-C₁₀-Aryl, welches durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
C₆-C₁₀-Aryl-C₁-C₄-alkylen, welches am Arylrest gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl, welches ein- oder mehrfach durch Methyl, Halogen, Hydroxy, CF₃ oder Methoxy substituiert sein kann, oder
C₆-C₁₀-Aryl-C₁-C₄-alkylen, welches am Arylrest durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
C₆-C₁₀-Aryl-C₁-C₄-alkylen, welches an der Alkylengruppe gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl und Phenyl, oder
ein 5 oder 6-gliedriger gesättigter oder ungesättigter Ring, der ein, zwei oder drei Heteroatome ausgewählt aus der Gruppe bestehend aus Stickstoff, Sauerstoff oder Schwefel enthalten kann und der gegebenenfalls ein- oder mehrfach substituiert sein kann durch einen oder mehrere Reste ausgewählt aus der Gruppe bestehend aus C₁-C₄-Alkyl Hydroxy, Halogen, CF₃, Phenyl, Benzyl und -OC₁-C₄-Alkyl, oder
ein 5 oder 6-gliedriger gesättigter oder ungesättigter Ring, der ein, zwei oder drei Heteroatome ausgewählt aus der Gruppe bestehend aus Stickstoff, Sauerstoff oder Schwefel enthalten kann und der durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
C₃-C₆-Cycloalkyl, welches gegebenenfalls substituiert sein kann durch einen oder mehrere Reste ausgewählt aus der Gruppe bestehend aus C₁-C₄-Alkyl, Hydroxy, Halogen, CF₃, -OC₁-C₄-Alkyl, Phenyl und Phenyl, welches ein- oder mehrfach durch Methyl, Halogen, Hydroxy, CF₃ oder Methoxy substituiert sein kann, oder
C₃-C₆-Cycloalkyl, das durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann, oder
ein Rest der Formel 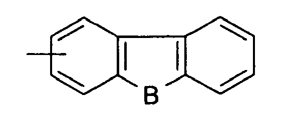 worin B -CH₂-, -NH-, -S- oder -O- bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 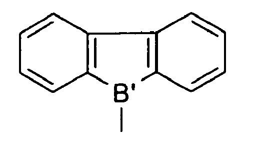 worin B' CH oder N bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 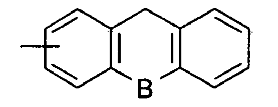 worin B -CH₂-, -NH-, -S- oder -O- bedeutet, der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
ein Rest der Formel 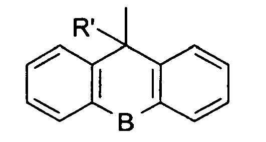 worin B -CH₂-, -NH-, -S- oder -O- bedeutet,
R' für Wasserstoff, Hydroxy, Methyl, Hydroxymethyl, Ethyl, -CF₃, CHF₂ oder Halogen stehen kann, und der gegebenenfalls ein- oder mehrfach substituiert sein kann durch einen oder mehrere Reste ausgewählt aus der Gruppe bestehend aus aus C₁-C₄-Alkyl, Hydroxy, Halogen, CF₃ und -OC₁-C₄-Alkyl, oder
R³ und R⁴ bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann und der gegebenenfalls ein- oder mehrfach substituiert sein kann durch einen oder mehrere Reste ausgewählt aus der Gruppe bestehend aus C₁-C₄-Alkyl Hydroxy, Halogen, CF₃, Phenyl, Benzyl und -OC₁-C₄-Alkyl, oder
R³ und R⁴ bilden gemeinsam mit dem Stickstoffatom einen 5- oder 6-gliedrigen gesättigten oder ungesättigten heterocyclischen Ring, der durch einen 5- oder 6-gliedrigen Heteroarylring substituiert ist, der gegebenenfalls ein- oder mehrfach durch Methyl, Halogen, Hydroxy, CF₃ oder Methoxy substituiert sein kann,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

2. Verbindungen der allgemeinen Formel **1** nach Anspruch 1, worin
X⁻ ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat ist.

3. Verbindungen der allgemeinen Formel 1 nach einem der Ansprüche 1 oder 2, worin
A ein zweibindiger Rest ausgewählt aus der Gruppe bestehend aus 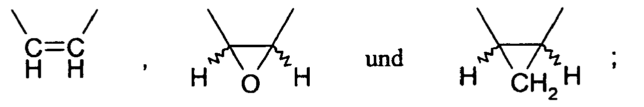
X⁻ ein ein Anion ausgewählt aus der Gruppe bestehend aus Chlorid, Bromid, Methansulfonat und p-Toluolsulfonat, bevorzugt Bromid;
R¹ und R² gleich oder verschieden, C₁-C₃-Alkyl, welches gegebenenfalls durch einen Rest ausgewählt aus der Gruppe bestehend aus C₃-C₅-Cycloalkyl, Hydroxy, oder Fluor substituiert sein kann,
oder
R¹ und R² gemeinsam eine C₃-C₄-Alkylen-Brücke;
R³ und R⁴ gleich oder verschieden,
Wasserstoff, oder
C₁-C₅-Alkyl, welches gegebenenfalls substituiert sein kann durch einen Rest ausgewählt aus der Gruppe bestehend aus Hydroxy, Fluor, CF₃ und Methoxy, oder
ein Phenyl- oder Naphthylrest, welcher gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Phenyl- oder Naphthylrest, welcher durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Benzyl- oder Phenylethylrest, welcher am Phenylring gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Benzyl- oder Phenylethylrest, welcher am Phenylring durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Benzyl- oder Phenylethylrest, welcher an der Alkylenbrücke gegebenenfalls substituiert sein kann durch einen oder zwei, bevorzugt einen Rest ausgewählt aus der Gruppe bestehend aus Methyl, Ethyl, Hydroxy, Fluor, Chlor, Brom, CF₃, Methoxy und Phenyl, oder
ein 5, oder 6- gliedriger gesättigter oder ungesättigter Ring, der ein, zwei oder drei Heteroatome ausgewählt aus der Gruppe bestehend aus Stickstoff, Sauerstoff oder Schwefel enthalten kann und der gegebenenfalls ein-, zwei- oder dreifach substituiert sein kann durch einen oder mehrere Reste ausgewählt aus der Gruppe bestehend aus Methyl, Ethyl, Hydroxy, Fluor, Chlor, Brom, CF₃, Phenyl, Benzyl und Methoxy, oder
ein 5, oder 6- gliedriger gesättigter oder ungesättigter Ring, der ein, zwei oder drei Heteroatome ausgewählt aus der Gruppe bestehend aus Stickstoff, Sauerstoff oder Schwefel enthalten kann und der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Cyclopentyl- oder Cyclohexylrest, welcher gegebenenfalls substituiert sein kann durch einen, zwei oder drei Reste ausgewählt aus der Gruppe bestehend aus Methyl, Ethyl, Hydroxy, Fluor, Chlor, Brom, CF₃, Methoxy, Phenyl und Phenyl welches ein-, zwei- oder dreifach durch Methyl, Fluor, Chlor, Brom, Hydroxy, CF₃ oder Methoxy substituiert sein kann, oder
ein Cyclopentyl- oder Cyclohexylrest, der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann, oder
ein Rest der Formel 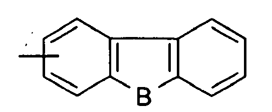 worin B -CH₂-, -NH-, -S- oder -O- bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 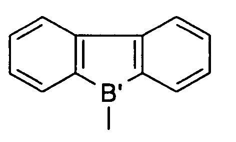 worin B' CH oder N bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 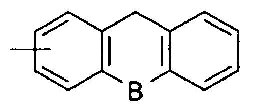 worin B -CH₂-, -NH-, -S- oder -O- bedeutet, der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
ein Rest der Formel 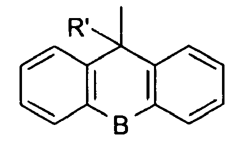 worin B -CH₂-, -NH-, -S- oder -O- bedeutet,
R' für Wasserstoff, Hydroxy, Methyl, Hydroxymethyl, Ethyl, -CF₃, CHF₂ oder Fluor stehen kann, und der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, CF₃ oder Methoxy, oder
R³ und R⁴ bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann und der gegebenenfalls ein-, zwei- oder dreifach substituiert sein kann durch einen oder mehrere Reste ausgewählt aus der Gruppe bestehend aus Methyl, Fluor, Chlor, Brom, Hydroxy, Phenyl, CF₃ oder Methoxy, oder
R³ und R⁴ bilden gemeinsam mit dem Stickstoffatom einen 5- oder 6-gliedrigen gesättigten oder ungesättigten heterocyclischen Ring, der gegebenenfalls ein oder zwei weitere Heteroatome ausgewählt aus der Gruppe bestehend aus Stickstoff, Sauerstoff oder Schwefel enthalten kann, der durch einen Heteroarylring ausgewählt aus der Gruppe bestehend aus Furan, Thiophen, Pyrrol, Imidazol, Pyridin und Pyrimidin substituiert ist, der gegebenenfalls ein- oder zweifach durch Methyl, Fluor, Chlor Brom, Hydroxy, CF₃ oder Methoxy substituiert sein kann,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

4. Verbindungen der allgemeinen Formel **1** gemäß einem der Ansprüche 1 bis 3, worin
A ein zweibindiger Rest ausgewählt aus der Gruppe bestehend aus 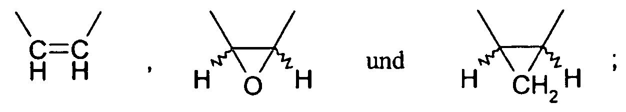
X⁻ ein Anion ausgewählt aus der Gruppe bestehend aus Chlorid, Bromid, Methansulfonat und p-Toluolsulfonat, bevorzugt Bromid;
R¹ und R² gleich oder verschieden, ein Methyl- oder Ethylrest, der gegebenenfalls durch Cyclopropyl, Hydroxy oder Fluor substituiert sein kann,
oder
R¹ und R² gemeinsam eine C₃-C₄-Alkylen-Brücke;
R³ Wasserstoff oder C₁-C₃-Alkyl, welches gegebenenfalls substituiert sein kann durch einen Rest ausgewählt aus der Gruppe bestehend aus Hydroxy, Fluor oder CF₃;
R⁴ C₁-C₃-Alkyl, welches gegebenenfalls substituiert sein kann durch einen Rest ausgewählt aus der Gruppe bestehend aus Hydroxy, Fluor oder CF₃;
R⁴ ein Phenylrest, der gegebenenfalls substituiert sein kann durch einen oder zwei, bevorzugt einen Rest ausgewählt aus der Gruppe bestehend aus Furyl, Thienyl, Phenyl und Phenyl welches ein-, zwei- oder dreifach durch Methyl, Fluor, Chlor, Brom, Hydroxy, CF₃ oder Methoxy substituiert sein kann, oder
R⁴ ein Benzylrest, welcher am Phenylring gegebenenfalls substituiert sein kann durch einen, zwei oder drei, bevorzugt einen Rest ausgewählt aus der Gruppe bestehend aus Methyl, Ethyl, Hydroxy, Fluor, Chlor, Brom, CF₃, Methoxy, Furyl, Thienyl und Phenyl, oder
R⁴ ein Benzylrest, welcher an der Methylenbrücke gegebenenfalls substituiert sein kann durch einen, zwei oder drei, bevorzugt einen Rest ausgewählt aus der Gruppe bestehend aus Methyl, Ethyl, Hydroxy, Fluor, Chlor, Brom, CF₃, Methoxy und Phenyl, oder
R⁴ ein Rest der Formel 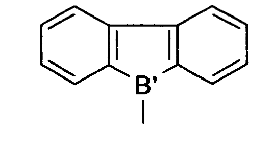 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

5. Verbindungen der allgemeinen Formel 1 gemäß einem der Ansprüche 1 bis 4, worin
A ein zweibindiger Rest ausgewählt aus der Gruppe bestehend aus 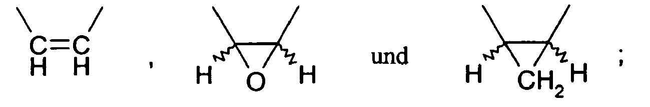
X⁻ ein einfach negativ geladenes Anion ausgewählt aus der Gruppe bestehend aus Chlorid, Bromid, Methansulfonat und p-Toluolsulfonat, bevorzugt Bromid;
R¹ und R² gleich oder verschieden, ein Methyl- oder Ethylrest, der gegebenenfalls durch Cyclopropyl, Hydroxy oder Fluor substituiert sein kann,
oder
R¹ und R² gemeinsam eine C₃-C₄-Alkylen-Brücke;
R³ Wasserstoff oder C₁-C₃-Alkyl, welches gegebenenfalls substituiert sein kann durch einen Rest ausgewählt aus der Gruppe bestehend aus Hydroxy, Fluor oder CF₃;
R⁴ C₁-C₃-Alkyl, welches gegebenenfalls substituiert sein kann durch einen Rest ausgewählt aus der Gruppe bestehend aus Hydroxy, Fluor oder CF₃;
R⁴ ein Phenylrest, der gegebenenfalls substituiert sein kann durch Phenyl, welches gegebenenfalls ein- oder zweifach durch Methyl, Fluor, Hydroxy oder CF₃ substituiert sein kann, oder
R⁴ ein Benzylrest, welcher am Phenylring gegebenenfalls substituiert sein kann durch einen oder zwei, bevorzugt einen Rest ausgewählt aus der Gruppe bestehend aus Methyl, Ethyl, Hydroxy, Fluor, CF₃, und Phenyl, oder
R⁴ ein Benzylrest, welcher an der Methylenbrücke gegebenenfalls einfach durch Phenyl substituiert sein kann, oder
R⁴ ein Rest der Formel 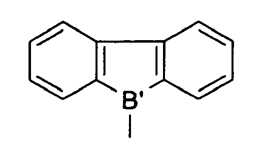 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

6. Verbindungen der allgemeinen Formel **1** gemäß einem der Ansprüche 1 bis 5, worin X⁻ Bromid oder Methansulfonat bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

7. Verbindungen der allgemeinen Formel **1** gemäß einem der Ansprüche 1 bis 6, worin R¹ und R² Methyl bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

8. Verbindungen der allgemeinen Formel **1** gemäß einem der Ansprüche 1 bis 7, worin R³ Wasserstoff oder Methyl bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

9. Verbindungen der allgemeinen Formel **1** gemäß einem der Ansprüche 1 bis 8, worin R⁴ Biphenyl, Benzhydryl, Fluorenyl oder Biphenylmethyl bedeutet, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer pharmakologisch verträglichen Säureadditionssalze, Solvate und Hydrate.

10. Verbindung der allgemeinen Formel **1** gemäß einem der Ansprüche 1 bis 9 als Arzneimittel.

11. Verwendung einer Verbindung der allgemeinen Formel **1** gemäß einem der Ansprüche 1 bis 9 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

12. Pharmazeutische Zubereitungen, enthaltend als Wirkstoff eine oder mehrere Verbindungen der allgemeinen Formel **1** gemäß einem der Ansprüche 1 bis 9 oder deren physiologisch verträgliche Salze gegebenenfalls in Kombination mit üblichen Hilfs- und/oder Trägerstoffen.

13. Pharmazeutische Zubereitungen nach Anspruch 12, **dadurch gekennzeichnet daß** diese neben einer oder mehrerer der Verbindungen der Formel **1** ferner wenigstens einen weiteren Wirkstoff enthalten, der ausgewählt ist aus der Gruppe der Betamimetica, Antiallergika, PAF-Antagonisten, PDE IV-Inhibitoren, Leukotrien-Antagonisten, p38 Kinase-Inhibitoren, EGFR-Kinase-Hemmern und Corticosteroiden.

14. Zwischenprodukte der Formel **4** 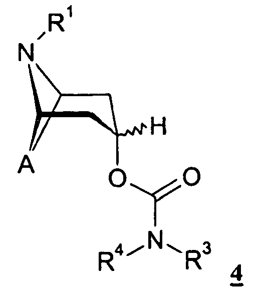 worin
A ein zweibindiger Rest ausgewählt aus der Gruppe bestehend aus 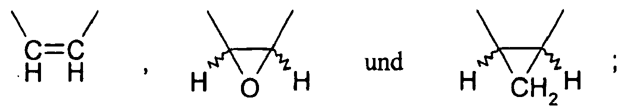
R¹ ein Methyl- oder Ethylrest, der gegebenenfalls durch Cyclopropyl, Hydroxy oder Fluor substituiert sein kann, oder
R¹ C₃-C₄-Alkylen-X, wobei X Chlorid, Bromid, Methansulfonat und p-Toluolsulfonat;
R³ Wasserstoff oder C₁-C₃-Alkyl, welches gegebenenfalls substituiert sein kann durch einen Rest ausgewählt aus der Gruppe bestehend aus Hydroxy, Fluor oder CF₃;
R⁴ C₁-C₃-Alkyl, welches gegebenenfalls substituiert sein kann durch einen Rest ausgewählt aus der Gruppe bestehend aus Hydroxy, Fluor oder CF₃, oder
R⁴ ein Phenylrest, der substituiert ist durch Phenyl, welches gegebenenfalls ein- oder zweifach durch Methyl, Fluor, Hydroxy oder CF₃ substituiert sein kann, oder
R⁴ ein Benzylrest, welcher am Phenylring gegebenenfalls substituiert sein kann durch einen oder zwei, bevorzugt einen Rest ausgewählt aus der Gruppe bestehend aus Methyl, Ethyl, Hydroxy, Fluor, CF₃, und Phenyl, oder
R⁴ ein Benzylrest, welcher an der Methylenbrücke gegebenenfalls einfach durch Phenyl substituiert sein kann, oder
R⁴ ein Rest der Formel 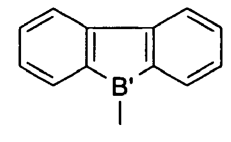 worin B' CH bedeutet, der gegebenenfalls ein- oder zweifach substituiert sein kann durch einen oder mehrere Reste ausgewählt aus der Gruppe bestehend aus Methyl, Fluor, Chlor, Brom, Hydroxy, CF₃ oder Methoxy,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer Säureadditionssalze, Solvate und Hydrate.

15. Zwischenprodukte der Formel **4** gemäß Anspruch 14,
worin
A ein zweibindiger Rest ausgewählt aus der Gruppe bestehend aus 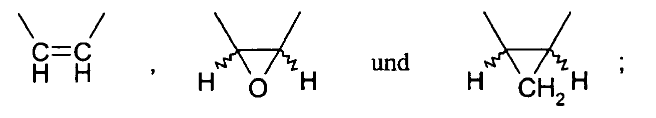
R¹ Methyl;
R³ Wasserstoff oder Methyl;
R⁴ Biphenyl, Benzhydryl, Fluorenyl oder Biphenylmethyl,
bedeuten, gegebenenfalls in Form der einzelnen Enantiomere oder Diasteromere, Mischungen der jeweiligen Enantiomere oder Diastereomere sowie gegebenenfalls in Form der Racemate, sowie gegebenenfalls in Form ihrer Säureadditionssalze, Solvate und Hydrate.

## Claims

1. Compounds of general formula **1** 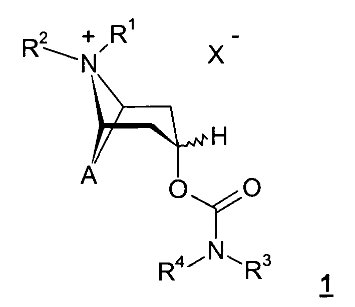 wherein
A denotes a double-bonded group selected from among 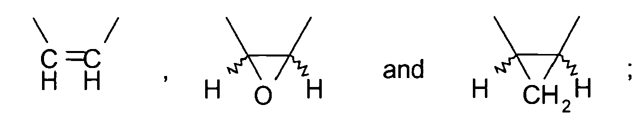
X⁻ denotes an anion with a single negative charge;
R¹ and R² which may be identical or different, denote C₁-C₅-alkyl, which may optionally be substituted by a group selected from the group consisting of -C₃-C₆-cycloalkyl, hydroxy or halogen,
or
R¹ and R² together denote a C₃-C₅-alkylene bridge;
R³ and R⁴ which may be identical or different, denote
hydrogen, or
C₁-C₅-alkyl, which may optionally be mono- or polysubstituted by one or more groups selected from the group consisting of hydroxy, halogen, CF₃ and -OC₁-C₄-alkyl, or
a C₂-C₅-alkenyl or C₂-C₅-alkynyl group, which may optionally be mono- or polysubstituted by one or more groups selected from the group consisting of hydroxy, halogen, CF₃, -OC₁-C₄-alkyl, phenyl and phenyl which may be mono- or polysubstituted by methyl, halogen, hydroxy, CF₃ or methoxy, or
C₆-C₁₀-aryl, which may optionally be substituted by one or more groups selected from the group consisting of C₁-C₄-alkyl, hydroxy, halogen, CF₃, -OC₁-C₄-alkyl, phenyl and phenyl which may be mono- or polysubstituted by methyl, halogen, hydroxy, CF₃ or methoxy, or
C₆-C₁₀-aryl, which is substituted by a 5- or 6-membered heteroaryl ring, which may optionally be mono- or polysubstituted by methyl, halogen, hydroxy, CF₃ or methoxy, or
C₆-C₁₀-aryl-C₁-C₄-alkylene, which may optionally be substituted at the aryl group by one or more groups selected from the group consisting of C₁-C₄-alkyl, hydroxy, halogen, CF₃, -OC₁-C₄-alkyl, phenyl and phenyl which may be mono- or polysubstituted by methyl, halogen, hydroxy, CF₃ or methoxy, or
C₆-C₁₀-aryl-C₁-C₄-alkylene, which is substituted at the aryl group by a 5- or 6-membered heteroaryl ring, which may optionally be mono- or polysubstituted by methyl, halogen, hydroxy, CF₃ or methoxy, or
C₆-C₁₀-aryl-C₁-C₄-alkylene, which may optionally be substituted at the alkylene group by one or more groups selected from the group consisting of C₁-C₄-alkyl, hydroxy, halogen, CF₃, -OC₁-C₄-alkyl and phenyl, or
a 5- or 6-membered saturated or unsaturated ring which may contain one, two or three heteroatoms selected from the group consisting of nitrogen, oxygen or sulphur and which may optionally be mono- or polysubstituted by one or more groups selected from the group consisting of C₁-C₄-alkyl hydroxy, halogen, CF₃, phenyl, benzyl and -OC₁-C₄-alkyl, or
a 5- or 6-membered saturated or unsaturated ring which may contain one, two or three heteroatoms selected from the group consisting of nitrogen, oxygen or sulphur and which is substituted by a 5- or 6-membered heteroaryl ring, which may optionally be mono- or polysubstituted by methyl, halogen, hydroxy, CF₃ or methoxy, or
C₃-C₆-cycloalkyl, which may optionally be substituted by one or more groups selected from the group consisting of C₁-C₄-alkyl, hydroxy, halogen, CF₃, -OC₁-C₄-alkyl, phenyl and phenyl which may be mono- or polysubstituted by methyl, halogen, hydroxy, CF₃ or methoxy, or
C₃-C₆-cycloalkyl, which is substituted by a 5- or 6-membered heteroaryl ring, which may optionally be mono- or polysubstituted by methyl, halogen, hydroxy, CF₃ or methoxy, or
a group of formula 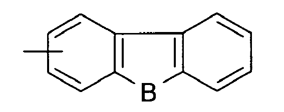 wherein B denotes -CH₂, -NH, -S or -O-, which may optionally be mono- or polysubstituted by one or more groups selected from the group consisting of C₁-C₄-alkyl, hydroxy, halogen, CF₃ and -OC₁-C₄-alkyl, or
a group of formula 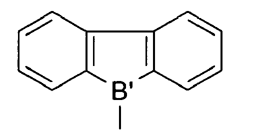 wherein B' denotes CH or N, which may optionally be mono- or polysubstituted by one or more groups selected from the group consisting of C₁-C₄-alkyl, hydroxy, halogen, CF₃ and -OC₁-C₄-alkyl, or
a group of formula 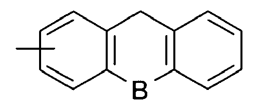 wherein B denotes -CH₂, -NH, -S or -O-, which may optionally be mono- or polysubstituted by one or more groups selected from the group consisting of C₁-C₄-alkyl, hydroxy, halogen, CF₃ and -OC₁-C₄-alkyl, or
a group of formula 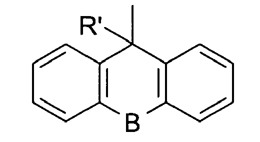 wherein B denotes -CH₂, -NH, -S or -O-,
R' may represent hydrogen, hydroxy, methyl, hydroxymethyl, ethyl, -CF₃, CHF₂ or halogen, and which may optionally be mono- or polysubstituted by one or more groups selected from the group consisting of C₁-C₄-alkyl, hydroxy, halogen, CF₃ and -OC₁-C₄-alkyl, or
R³ and R⁴ together with the nitrogen atom form a 5- or 6-membered saturated or unsaturated heterocyclic ring which may optionally contain one or two more heteroatoms selected from the group consisting of nitrogen, oxygen or sulphur and which may optionally be mono- or polysubstituted by one or more groups selected from the group consisting of C₁-C₄-alkyl hydroxy, halogen, CF₃, phenyl, benzyl and -OC₁-C₄-alkyl, or
R³ and R⁴ together with the nitrogen atom form a 5- or 6-membered saturated or unsaturated heterocyclic ring which is substituted by a 5- or 6-membered heteroaryl ring, which may optionally be mono- or polysubstituted by methyl, halogen, hydroxy, CF₃ or methoxy,
optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

2. Compounds of general formula **1,** wherein
X⁻ denotes an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate.

3. Compounds of general formula **1** according to one of claims 1 or 2, wherein
A denotes a double-bonded group selected from among 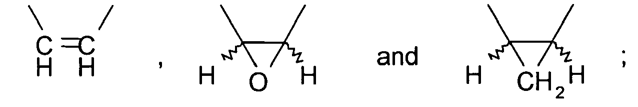
X⁻ denotes an anion selected from the group consisting of chloride, bromide, methanesulphonate and p-toluenesulphonate, preferably bromide;
R¹ and R² which may be identical or different, denote C₁-C₃-alkyl, which may optionally be substituted by a group selected from the group consisting of C₃-C₅-cycloalkyl, hydroxy, or fluorine,
or
R¹ and R² together denote a C₃-C₄-alkylene bridge;
R³ and R⁴ which may be identical or different, denote
hydrogen, or
C₁-C₅-alkyl, which may optionally be substituted by a group selected from the group consisting of hydroxy, fluorine, CF₃ and methoxy, or
a phenyl or naphthyl group which may optionally be substituted by one, two or three groups selected from the group consisting of methyl, ethyl, hydroxy, fluorine, chlorine, bromine, CF₃, methoxy, phenyl and phenyl which may be mono-, di- or trisubstituted by methyl, fluorine, chlorine, bromine, hydroxy, CF₃ or methoxy, or
a phenyl or naphthyl group which is substituted by a heteroaryl ring selected from the group consisting of furan, thiophene, pyrrole, imidazole, pyridine and pyrimidine, which may optionally be mono- or disubstituted by methyl, fluorine, chlorine bromine, hydroxy, CF₃ or methoxy, or
a benzyl or phenylethyl group which may optionally be substituted at the phenyl ring by one, two or three groups selected from the group consisting of methyl, ethyl, hydroxy, fluorine, chlorine, bromine, CF₃, methoxy, phenyl and phenyl which may be mono-, di- or trisubstituted by methyl, fluorine, chlorine, bromine, hydroxy, CF₃ or methoxy, or
a benzyl or phenylethyl group which is substituted at the phenyl ring by a heteroaryl ring selected from the group consisting of furan, thiophene, pyrrole, imidazole, pyridine and pyrimidine, which may optionally be mono- or disubstituted by methyl, fluorine, chlorine bromine, hydroxy, CF₃ or methoxy, or
a benzyl or phenylethyl group which may optionally be substituted at the alkylene bridge by one or two, preferably one group selected from the group consisting of methyl, ethyl, hydroxy, fluorine, chlorine, bromine, CF₃, methoxy and phenyl, or
a 5- or 6- membered saturated or unsaturated ring which may contain one, two or three heteroatoms selected from the group consisting of nitrogen, oxygen or sulphur and which may optionally be mono-, di- or trisubstituted by one or more groups selected from the group consisting of methyl, ethyl, hydroxy, fluorine, chlorine, bromine, CF₃, phenyl, benzyl and methoxy, or
a 5- or 6- membered saturated or unsaturated ring which may contain one, two or three heteroatoms selected from the group consisting of nitrogen, oxygen or sulphur and which is substituted by a heteroaryl ring selected from the group consisting of furan, thiophene, pyrrole, imidazole, pyridine and pyrimidine, which may optionally be mono- or disubstituted by methyl, fluorine, chlorine bromine, hydroxy, CF₃ or methoxy, or
a cyclopentyl or cyclohexyl group which may optionally be substituted by one, two or three groups selected from the group consisting of methyl, ethyl, hydroxy, fluorine, chlorine, bromine, CF₃, methoxy, phenyl and phenyl which may be mono-, di- or trisubstituted by methyl, fluorine, chlorine, bromine, hydroxy, CF₃ or methoxy, or
a cyclopentyl or cyclohexyl group which is substituted by a heteroaryl ring selected from the group consisting of furan, thiophene, pyrrole, imidazole, pyridine and pyrimidine, which may optionally be mono- or disubstituted by methyl, fluorine, chlorine bromine, hydroxy, CF₃ or methoxy, or
a group of formula 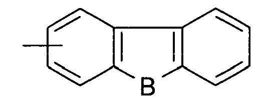 wherein B denotes - CH₂, -NH, -S or -O-, which may optionally be mono-, di- or trisubstituted by one or more groups selected from the group consisting of methyl, fluorine, chlorine, bromine, hydroxy, CF₃ or methoxy, or
a group of formula 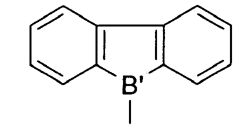 wherein B' denotes CH or N, which may optionally be mono-, di- or trisubstituted by one or more groups selected from the group consisting of methyl, fluorine, chlorine, bromine, hydroxy, CF₃ or methoxy, or
a group of formula 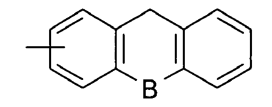 wherein B denotes -CH₂, -NH, -S or -O-, which may optionally be mono-, di- or trisubstituted by one or more groups selected from the group consisting of methyl, fluorine, chlorine, bromine, hydroxy, CF₃ or methoxy, or
a group of formula 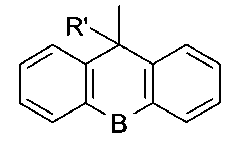 wherein B denotes -CH₂, -NH, -S or -O-,
R' may represent hydrogen, hydroxy, methyl, hydroxymethyl, ethyl, -CF₃, CHF₂ or fluorine, and which may optionally be mono-, di- or trisubstituted by one or more groups selected from the group consisting of methyl, fluorine, chlorine, bromine, hydroxy, CF₃ or methoxy, or
R³ and R⁴ together with the nitrogen atom form a 5- or 6-membered saturated or unsaturated heterocyclic ring which may optionally contain one or two more heteroatoms selected from the group consisting of nitrogen, oxygen or sulphur and which may optionally be mono-, di- or trisubstituted by one or more groups selected from the group consisting of methyl, fluorine, chlorine, bromine, hydroxy, phenyl, CF₃ or methoxy, or
R³ and R⁴ together with the nitrogen atom form a 5- or 6-membered saturated or unsaturated heterocyclic ring which may optionally contain one or two more heteroatoms selected from the group consisting of nitrogen, oxygen or sulphur, which is substituted by a heteroaryl ring selected from the group consisting of furan, thiophene, pyrrole, imidazole, pyridine and pyrimidine, which may optionally be mono- or disubstituted by methyl, fluorine, chlorine bromine, hydroxy, CF₃ or methoxy,
optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

4. Compounds of general formula **1** according to one of claims 1 to 3,
wherein
A denotes a double-bonded group selected from among 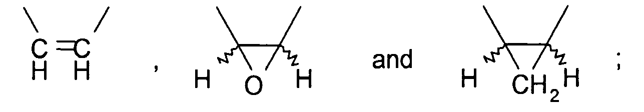
X⁻ denotes an anion selected from the group consisting of chloride, bromide, methanesulphonate and p-toluenesulphonate, preferably bromide;
R¹ and R² which may be identical or different, denote a methyl or ethyl group which may optionally be substituted by cyclopropyl, hydroxy or fluorine,
or
R¹ and R² together denote a C₃-C₄-alkylene bridge;
R³ denotes hydrogen or C₁-C₃-alkyl, which may optionally be substituted by a group selected from the group consisting of hydroxy, fluorine or CF₃;
R⁴ denotes C₁-C₃-alkyl, which may optionally be substituted by a group selected from the group consisting of hydroxy, fluorine or CF₃;
R⁴ denotes a phenyl group which may optionally be substituted by one or two, preferably one group selected from the group consisting of furyl, thienyl, phenyl and phenyl which may be mono-, di- or trisubstituted by methyl, fluorine, chlorine, bromine, hydroxy, CF₃ or methoxy, or
R⁴ denotes a benzyl group which may optionally be substituted at the phenyl ring by one, two or three, preferably one group selected from the group consisting of methyl, ethyl, hydroxy, fluorine, chlorine, bromine, CF₃, methoxy, furyl, thienyl and phenyl, or
R⁴ denotes a benzyl group which may optionally be substituted at the methylene bridge by one, two or three, preferably one group selected from the group consisting of methyl, ethyl, hydroxy, fluorine, chlorine, bromine, CF₃, methoxy and phenyl, or
R⁴ denotes a group of formula 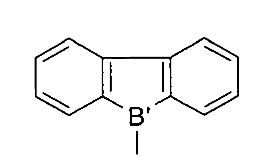 wherein B' denotes CH, which may optionally be mono- or disubstituted by one or more groups selected from the group consisting of methyl, fluorine, chlorine, bromine, hydroxy, CF₃ or methoxy,
optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

5. Compounds of general formula **1** according to one of claims 1 to 4, wherein
A denotes a double-bonded group selected from among 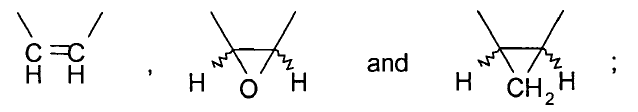
X⁻ denotes an anion with a single negative charge selected from the group consisting of chloride, bromide, methanesulphonate and p-toluenesulphonate, preferably bromide;
R¹ and R² which may be identical or different, denote a methyl or ethyl group which may optionally be substituted by cyclopropyl, hydroxy or fluorine,
or
R¹ and R² together denote a C₃-C₄-alkylene bridge;
R³ denotes hydrogen or C₁-C₃-alkyl, which may optionally be substituted by a group selected from the group consisting of hydroxy, fluorine or CF₃;
R⁴ denotes C₁-C₃-alkyl, which may optionally be substituted by a group selected from the group consisting of hydroxy, fluorine or CF₃;
R⁴ denotes a phenyl group which may optionally be substituted by phenyl, which may optionally be mono- or disubstituted by methyl, fluorine, hydroxy or CF₃, or
R⁴ denotes a benzyl group which may optionally be substituted at the phenyl ring by one or two, preferably one group selected from the group consisting of methyl, ethyl, hydroxy, fluorine, CF₃, and phenyl, or
R⁴ denotes a benzyl group which may optionally be monosubstituted at the methylene bridge by phenyl, or
R⁴ denotes a group of formula 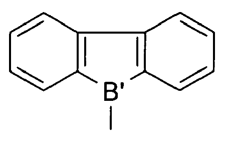 wherein B' denotes CH, which may optionally be mono- or disubstituted by one or more groups selected from the group consisting of methyl, fluorine, chlorine, bromine, hydroxy, CF₃ or methoxy,
optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

6. Compounds of general formula **1** according to one of claims 1 to 5, wherein X⁻ denotes bromide or methanesulphonate, optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

7. Compounds of general formula **1** according to one of claims 1 to 6,
wherein R¹ and R² represent methyl, optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

8. Compounds of general formula **1** according to one of claims 1 to 7,
wherein R³ denotes hydrogen or methyl, optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

9. Compounds of general formula **1** according to one of claims 1 to 8,
wherein
R⁴ denotes biphenyl, benzhydryl, fluorenyl or biphenylmethyl, optionally in the form of the individual enantiomers or diastereomers, mixtures of the enantiomers or diastereomers and optionally in the form of the racemates, and optionally in the form of the pharmacologically acceptable acid addition salts, solvates and hydrates thereof.

10. Compound of general formula **1** according to one of claims 1 to 9, as a medicament.

11. Use of a compound of general formula **1** according to one of claims 1 to 9 for preparing a medicament for the treatment of asthma, COPD, vagally induced sinus bradycardia, heart rhythm disorders, spasms in the gastrointestinal tract, spasms in the urinary tract and menstrual pain.

12. Pharmaceutical preparations, containing as active substance one or more compounds of general formula **1** according to one of claims 1 to 9 or the physiologically acceptable salts thereof optionally in combination with conventional excipients and/or carriers.

13. Pharmaceutical preparations according to claim 12, **characterised in that** they contain, in addition to one or more of the compounds of formula **1**, at least one other active substance which is selected from among the betamimetics, antiallergics, PAF antagonists, PDE IV inhibitors, leukotriene antagonists, p38 kinase inhibitors, EGFR kinase inhibitors and corticosteroids.

14. Intermediate products of formula **4** 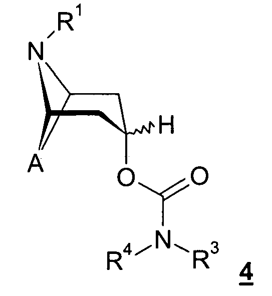 wherein
A denotes a double-bonded group selected from among 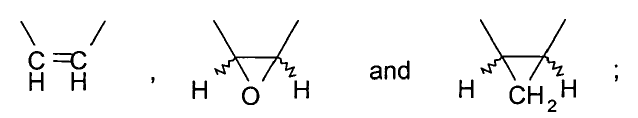
R¹ denotes a methyl or ethyl group which may optionally be substituted by cyclopropyl, hydroxy or fluorine, or
R¹ denotes C₃-C₄-alkylene-X, wherein X may represent chloride, bromide, methanesulphonate or p-toluenesulphonate;
R³ denotes hydrogen or C₁-C₃-alkyl, which may optionally be substituted by a group selected from the group consisting of hydroxy, fluorine or CF₃;
R⁴ denotes C₁-C₃-alkyl, which may optionally be substituted by a group selected from the group consisting of hydroxy, fluorine or CF₃, or
R⁴ denotes a phenyl group which is substituted by phenyl which may optionally be mono- or disubstituted by methyl, fluorine, hydroxy or CF₃, or
R⁴ denotes a benzyl group which may optionally be substituted at the phenyl ring by one or two, preferably one group selected from the group consisting of methyl, ethyl, hydroxy, fluorine, CF₃ and phenyl, or
R⁴ denotes a benzyl group which may optionally be monosubstituted at the methylene bridge by phenyl, or
R⁴ denotes a group of formula 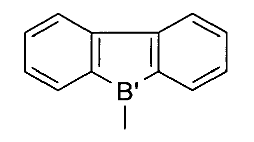 wherein B' denotes CH, which may optionally be mono- or disubstituted by one or more groups selected from the group consisting of methyl, fluorine, chlorine, bromine, hydroxy, CF₃ or methoxy,
optionally in the form of the individual enantiomers or diastereomers, mixtures of the enantiomers or diastereomers and optionally in the form of the racemates, and optionally in the form of the acid addition salts, solvates and hydrates thereof.

15. Intermediates of formula **4** according to claim 14,
worin
A denotes a double-bonded group selected from among 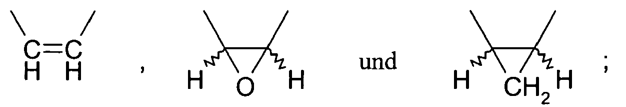
R¹ methyl;
R³ hydrogen oder methyl;
R⁴ denotes biphenyl, benzhydryl, fluorenyl or biphenylmethyl,
optionally in the form of the individual enantiomers or diastereomers, mixture: of the enantiomers or diastereomers and optionally in the form of the racemates, and optionally in the form of the acid addition salts, solvates and hydrates thereof.

## Revendications

1. Composés de formule générale 1 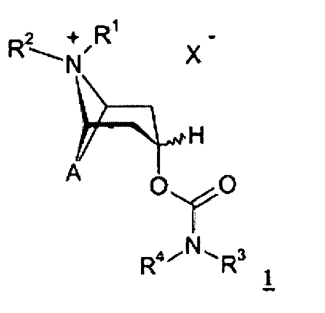 dans laquelle
A représente un radical à doubles liaisons, choisi dans le groupe constitué de : 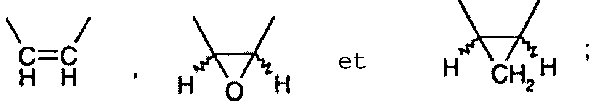
X⁻ représente un anion monochargé négativement;
R¹ et R² identiques ou différents, représentent alkyle en C₁ à C₅, qui peut être le cas échéant substitué par un radical choisi dans le groupe constitué par cycloalkyle en C₃ à C₆, hydroxy ou halogène,
ou
R¹ et R² ensemble, représentent un pont alkylène en C₃ à C₅ ;
R³ et R⁴ identiques ou différents, représentent un hydrogène, ou
alkyle en C₁ à C₅, qui peut être le cas échéant monosubstitué ou polysubstitué par un ou plusieurs radicaux choisis dans le groupe constitué par hydroxy, halogène, CF₃ et -O-(alkyle en C₁ à C₄), ou
un groupement alcényle en C₂ à C₅ ou alcinyle en C₂ à C₅, qui peut être le cas échéant monosubstitué ou polysubstitué par un ou plusieurs radicaux choisis dans le groupe constitué par hydroxy, halogène, CF₃, -O-(alkyle en C₁ à C₄), phényle et phényle qui peut être mono- ou polysubstitué par méthyle, halogène, hydroxy, CF₃ ou méthoxy, ou
aryle en C₆ à C₁₀, qui peut être le cas échéant substitué par un ou plusieurs radicaux choisis dans le groupe constitué par alkyle en C₁ à C₄, hydroxy, halogène, CF₃,-O-(alkyle en C₁ à C₄), phényle et phényle qui peut être mono- ou polysubstitué par méthyle, halogène, hydroxy, CF₃ ou méthoxy, ou
aryle en C₆ à C₁₀, qui est substitué par un cycle hétéroaryle de 5 ou 6 chaînons, qui peut être le cas échéant monosubstitué ou polysubstitué par méthyle, halogène, hydroxy, CF₃ ou méthoxy, ou
(aryle en C₆ à C₁₀)-(alkylène en C₁ à C₄), qui peut être le cas échéant substitué sur le radical aryle par un ou plusieurs radicaux choisis dans le groupe constitué par alkyle en C₁ à C₄, hydroxy, halogène, CF₃, -O-(alkyle en C₁ à C₄), phényle et phényle qui peut être mono- ou polysubstitué par méthyle, halogène, hydroxy, CF₃ ou méthoxy, ou
(aryle en C₆ à C₁₀)-(alkylène en C₁ à C₄), qui est substitué sur le radical aryle par un cycle hétéroaryle de 5 ou 6 chaînons, qui peut être le cas échéant monosubstitué ou polysubstitué par méthyle, halogène, hydroxy, CF₃ ou méthoxy, ou
(aryle en C₆ à C₁₀) - (alkylène en C₁ à C₄), qui peut être substitué le cas échéant sur le groupe alkylène par un ou plusieurs radicaux choisis dans le groupe constitué par alkyle en C₁ à C₄, hydroxy, halogène, CF₃, -O- (alkyle en C₁ à C₄), phényle, ou
un cycle saturé ou insaturé de 5 ou 6 chaînons, qui peut contenir un, deux ou trois hétéroatomes choisis dans le groupe constitué par azote, oxygène ou soufre, et qui peut être le cas échéant mono- ou polysubstitué par un ou plusieurs radicaux choisis dans le groupe constitué par alkyle en C₁ à C₄, hydroxy, halogène, CF₃, phényle, benzyle ou un -O-(alkyle en C₁ à C₄), ou
un cycle saturé ou insaturé de 5 ou 6 chaînons, qui peut contenir un, deux ou trois hétéroatomes choisis dans le groupe constitué par azote, oxygène ou soufre, et qui est substitué par un cycle hétéroaryle de 5 ou 6 chaînons, qui peut être le cas échéant mono- ou polysubstitué par méthyle, halogène, hydroxy, CF₃ ou méthoxy, ou
cycloalkyle en C₃ à C₆, qui peut être le cas échéant substitué par un ou plusieurs radicaux choisis dans le groupe constitué par alkyle en C₁ à C₄, hydroxy, halogène, CF₃, un -O-(alkyle en C₁ à C₄), phényle et phényle qui peut être mono- ou polysubstitué par méthyle, halogène, hydroxy, CF₃ ou méthoxy, ou
cycloalkyle en C₃ à C₆, qui est substitué par un cycle hétéroaryle de 5 ou 6 chaînons, qui peut être le cas échéant monosubstitué ou polysubstitué par méthyle, halogène, hydroxy, CF₃ ou méthoxy, ou
un radical de formule 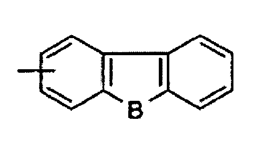 dans laquelle B représente -CH₂-, -NH-, -S- ou -O-, qui peut être le cas échéant mono- ou polysubstitué par un ou plusieurs radicaux choisis dans le groupe constitué par alkyle en C₁ à C₄, hydroxy, halogène, CF₃ et -O- (alkyle en C₁ à C₄), ou
un radical de formule 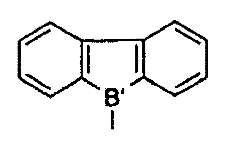 dans laquelle B' représente CH ou N, qui peut être le cas échéant mono- ou polysubstitué par un ou plusieurs radicaux choisis dans le groupe constitué par alkyle en C₁ à C₄, hydroxy, halogène, CF₃ et -O-(alkyle en C₁ à C₄), ou
un radical de formule 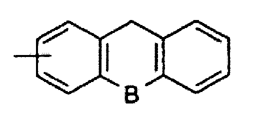 dans laquelle B représente -CH₂-, -NH-, -S- ou -O-, qui peut être le cas échéant mono- ou polysubstitué par un ou plusieurs radicaux choisis dans le groupe constitué par alkyle en C₁ à C₄, hydroxy, halogène, CF₃ et -O-(alkyle en C₁ à C₄), ou
un radical de formule
dans laquel 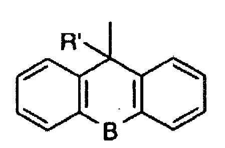 e -CH₂-, -NH-, -S- ou -O-,
R' peut representer hydrogène, hydroxy, méthyle, hydroxyméthyle, éthyle, -CF₃, CHF₂ ou halogène, et qui peut être le cas échéant mono- ou polysubstitué par un ou plusieurs radicaux choisis dans le groupe constitué par alkyle en C₁ à C₄, hydroxy, halogène, CF₃ et -O- (alkyle en C₁ à C₄), ou
R³ et R⁴ forment conjointement avec l'atome d'azote un cycle hétérocyclique saturé ou insaturé de 5 ou 6 chaînons, qui peut contenir le cas échéant un ou deux autres hétéroatomes choisis dans le groupe constitué par azote, oxygène ou soufre et qui peut être le cas échéant mono- ou polysubstitué par un ou plusieurs radicaux choisis dans le groupe constitué par alkyle en C₁ à C₄, hydroxy, halogène, CF₃, phényle, benzyle et -O-(alkyle en C₁ à C₄), ou
R³ et R⁴ forment conjointement avec l'atome d'azote un cycle hétérocyclique saturé ou insaturé de 5 ou 6 chaînons, qui est substitué par un cycle hétéroaryle de 5 ou 6 chaînons, qui peut être le cas échéant monosubstitué ou polysubstitué par méthyle, halogène, hydroxy, CF₃ ou méthoxy,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

2. Composés de formule générale 1 selon la revendication 1,
dans laquelle
X⁻ représente un anion choisi dans le groupe constitué par chlorure, bromure, iodure, sulfate, phosphate, méthanesulfonate, nitrate, maléate, acétate, citrate, fumarate, tartrate, oxalate, succinate, benzoate et p-toluènesulfonate .

3. Composés de formule générale 1 selon la revendication 1,
dans laquelle
A représente un radical à doubles liaisons, choisi dans le groupe constitué de : 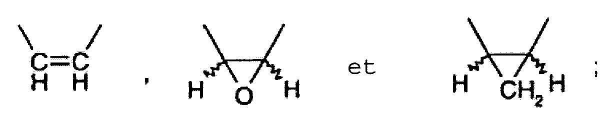
X⁻ représente un anion choisi dans le groupe constitué par chlorure, bromure, méthanesulfonate et p-toluènesulfonate, de préférence bromure ;
R¹ et R² identiques ou différents, représentent alkyle en C₁ à C₃, qui peut être le cas échéant substitué par un radical choisi dans le groupe constitué par cycloalkyle en C₃ à C₅, hydroxy ou fluor,
ou
R¹ et R² ensemble, représentent un pont alkylène en C₃ à C₄ ;
R³ et R⁴ identiques ou différents, représentent un hydrogène, ou
alkyle en C₁ à C₅, qui peut être le cas échéant substitué par un radical choisi dans le groupe constitué par hydroxy, fluor, CF₃ et méthoxy, ou
un radical phényle ou naphtyle, qui peut être le cas échéant substitué par un, deux ou trois radicaux choisis dans le groupe constitué par méthyle, éthyle, hydroxy, fluor, chlore, brome, CF₃, méthoxy, phényle et phényle qui peut être mono-, di- ou trisubstitué par méthyle, fluor, chlore, brome, hydroxy, CF₃ ou méthoxy, ou
un radical phényle ou naphtyle, qui est substitué par un cycle hétéroaryle, choisi dans le groupe constitué par furane, thiophène, pyrrole, imidazole, pyridine et pyrimidine, qui peut être le cas échéant mono- ou disubstitué par méthyle, fluor, chlore, brome, hydroxy, CF₃ ou méthoxy, ou
un radical benzyle ou phényléthyle, qui peut être le cas échéant substitué sur le cycle phényle par un, deux ou trois radicaux choisis dans le groupe constitué par méthyle, éthyle, hydroxy, fluor, chlore, brome, CF₃, méthoxy, phényle et phényle qui peut être mono-, di- ou trisubstitué par méthyle, fluor, chlore, brome, hydroxy, CF₃ ou méthoxy, ou
un radical benzyle ou phényléthyle, qui est substitué sur le cycle phényle par un cycle hétéroaryle, choisi dans le groupe constitué par furane, thiophène, pyrrole, imidazole, pyridine et pyrimidine, qui peut être le cas échéant mono- ou disubstitué par méthyle, fluor, chlore, brome, hydroxy, CF₃ ou méthoxy, ou
un radical benzyle ou phényléthyle, qui peut être le cas échéant substitué sur le pont alkylène par un ou deux radicaux, de préférence un, choisis dans le groupe constitué par méthyle, éthyle, hydroxy, fluor, chlore, brome, CF₃, méthoxy et phényle, ou
un cycle saturé ou insaturé de 5 ou 6 chaînons, qui peut contenir un, deux ou trois hétéroatomes choisis dans le groupe constitué par azote, oxygène ou soufre, et qui peut être le cas échéant mono-, di- ou trisubstitué par un ou plusieurs radicaux choisis dans le groupe constitué par méthyle, l'éthyle, hydroxy, fluor, chlore, brome, CF₃, phényle, le benzyle et méthoxy, ou
un cycle saturé ou insaturé de 5 ou 6 chaînons, qui peut contenir un, deux ou trois hétéroatomes choisis dans le groupe constitué par azote, oxygène ou soufre, et qui est substitué par un cycle hétéroaryle, choisi dans le groupe constitué par furane, thiophène, pyrrole, imidazole, pyridine et pyrimidine, qui peut être le cas échéant mono- ou disubstitué par méthyle, fluor, chlore, brome, hydroxy, le CF₃ ou méthoxy, ou
un radical cyclopentyle ou cyclohexyle, qui peut être substitué le cas échéant par un, deux ou trois radicaux choisis dans le groupe constitué par méthyle, éthyle, hydroxy, fluor, chlore, brome, CF₃, méthoxy, phényle et phényle qui peut être mono-, di- ou trisubstitué par méthyle, fluor, chlore, brome, hydroxy, CF₃ ou méthoxy, ou
un radical cyclopentyle ou cyclohexyle, qui est substitué par un cycle hétéroaryle, choisi dans le groupe constitué par furane, thiophène, pyrrole, imidazole, pyridine et pyrimidine, qui peut être le cas échéant mono- ou disubstitué par méthyle, fluor, chlore, brome, hydroxy, CF₃ ou méthoxy, ou
un radical de formule 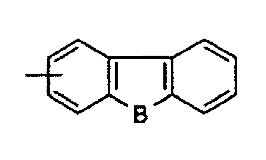 dans laquelle B représente -CH₂-, -NH-, -S- ou -O-, qui peut être le cas échéant mono-, di- ou trisubstitué par un ou plusieurs radicaux choisis dans le groupe constitué par méthyle, fluor, chlore, brome, hydroxy, CF₃ ou méthoxy, ou
un radical de formule 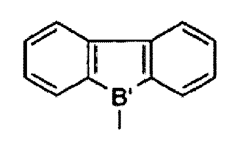 dans laquelle B' représente CH ou N, qui peut être le cas échéant mono-, di- ou trisubstitué par un ou plusieurs radicaux choisis dans le groupe constitué par méthyle, fluor, chlore, brome, hydroxy, CF₃ ou méthoxy, ou
un radical de formule 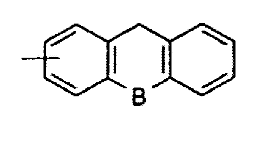 dans laquelle B représente -CH₂-, -NH-, -S- ou -O-, qui peut être le cas échéant mono-, di- ou trisubstitué par un ou plusieurs radicaux choisis dans le groupe constitué par méthyle, fluor, chlore, brome, hydroxy, CF₃ ou méthoxy, ou
un radical de formule 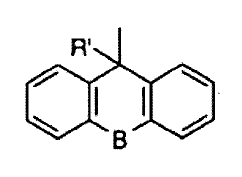 dans laquelle B représente -CH₂-, -NH-, -S- ou -O-,
R' peut représenter hydrogène, hydroxy, méthyle, hydroxyméthyle, éthyle, -CF₃, CHF₂ ou fluor, et qui peut être le cas échéant mono-, di- ou trisubstitué par un ou plusieurs radicaux choisis dans le groupe constitué par méthyle, fluor, chlore, brome, hydroxy, CF₃ ou méthoxy, ou
R³ et R⁴ forment conjointement avec l'atome d'azote un cycle hétérocyclique saturé ou insaturé de 5 ou 6 chaînons, qui peut contenir le cas échéant un ou deux autres hétéroatomes choisis dans le groupe constitué par azote, oxygène ou soufre et qui peut être le cas échéant mono-, di- ou trisubstitué par un ou plusieurs radicaux choisis dans le groupe constitué par méthyle, fluor, chlore, brome, hydroxy, phényle, CF₃ ou méthoxy, ou
R³ et R⁴ forment conjointement avec l'atome d'azote un cycle hétérocyclique saturé ou insaturé de 5 ou 6 chaînons, qui peut contenir le cas échéant un ou deux autres hétéroatomes choisis dans le groupe constitué par azote, oxygène ou soufre, qui est substitué par un cycle hétéroaryle, choisi dans le groupe constitué par furane, thiophène, pyrrole, imidazole, pyridine et pyrimidine, qui peut être le cas échéant mono- ou disubstitué par méthyle, fluor, chlore, brome, hydroxy, CF₃ ou méthoxy,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

4. Composés de formule générale 1 selon la revendication 1 ou la revendication 3,
dans laquelle
A représente un radical à doubles liaisons, choisi dans le groupe constitué de : 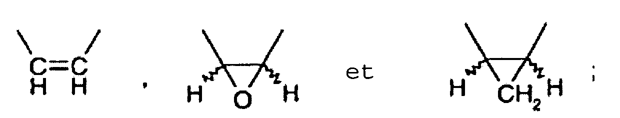
X⁻ représente un anion choisi dans le groupe constitué par chlorure, bromure, méthanesulfonate et p-toluènesulfonate, de préférence bromure ;
R¹ et R² identiques ou différents, représentent un radical méthyle ou éthyle, qui peut être le cas échéant substitué par cyclopropyle, hydroxy ou fluor,
ou
R¹ et R² ensemble, représentent un pont alkylène en C₃ à C₄ ;
R³ représente un hydrogène ou alkyle en C₁ à C₃, qui peut être le cas échéant substitué par un radical choisi dans le groupe constitué par hydroxy, fluor ou CF₃ ;
R⁴ représente alkyle en C₁ à C₃, qui peut être le cas échéant substitué par un radical choisi dans le groupe constitué par hydroxy, fluor ou CF₃ ;
R⁴ représente un radical phényle, qui peut être le cas échéant substitué par un ou deux radicaux, de préférence un radical choisi dans le groupe constitué par furyle, thiényle, phényle et phényle qui peut être mono-, di- ou trisubstitué par méthyle, fluor, chlore, brome, hydroxy, CF₃ ou méthoxy, ou
R⁴ représente un radical benzyle, qui peut être le cas échéant substitué sur le cycle phényle par un, deux ou trois radicaux, de préférence un radical choisi dans le groupe constitué par méthyle, éthyle, hydroxy, fluor, chlore, brome, CF₃, méthoxy, furyle, thiényle et phényle, ou
R⁴ représente un radical benzyle, qui peut être le cas échéant substitué sur le pont méthylène, par un, deux ou trois radicaux, de préférence un radical choisi dans le groupe constitué par méthyle, éthyle, hydroxy, fluor, chlore, brome, CF₃, méthoxy, et phényle, ou
R⁴ représente un radical de formule 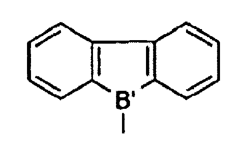 dans laquelle B' représente CH, qui peut être le cas échéant mono- ou disubstitué par un ou plusieurs radicaux choisis dans le groupe constitué par méthyle, fluor, chlore, brome, hydroxy, CF₃ ou méthoxy,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

5. Composés de formule générale 1 selon l'une des revendications 1 à 4,
dans laquelle
A représente un radical à doubles liaisons, choisi dans le groupe constitué de : 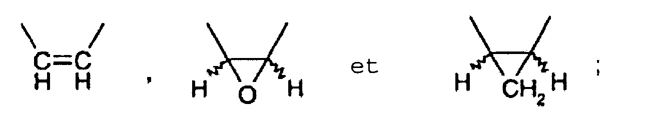
X⁻ représente un anion monochargé négativement, choisi dans le groupe constitué par chlorure, bromure, méthanesulfonate et p-toluènesulfonate, de préférence bromure ;
R¹ et R² identiques ou différents, représentent un radical méthyle ou éthyle, qui peut être le cas échéant substitué par cyclopropyle, hydroxy ou fluor,
ou
R¹ et R², ensemble, représentent un pont alkylène en C₃ à C₄ ;
R³ représente un hydrogène ou alkyle en C₁ à C₃, qui peut être le cas échéant substitué par un radical choisi dans le groupe constitué par hydroxy, fluor ou CF₃ ;
R⁴ représente alkyle en C₁ à C₃, qui peut être le cas échéant substitué par un radical choisi dans le groupe constitué par hydroxy, fluor ou CF₃ ;
R⁴ représente un radical phényle, qui peut être le cas échéant substitué par phényle, qui peut être le cas échéant mono- ou disubstitué par méthyle, fluor, hydroxy ou CF₃, ou
R⁴ représente un radical benzyle, qui peut être le cas échéant substitué sur le cycle phényle par un ou deux radicaux, de préférence un radical choisi dans le groupe constitué par méthyle, éthyle, hydroxy, fluor, CF₃ et phényle, ou
R⁴ représente un radical benzyle, qui peut être le cas échéant substitué sur le pont méthylène, par phényle, ou
R⁴ représente un radical de formule 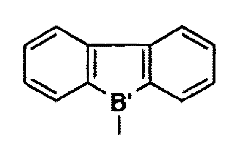 dans laquelle B' représente CH, qui peut être le cas échéant mono- ou disubstitué par un ou plusieurs radicaux choisis dans le groupe constitué par méthyle, fluor, chlore, brome, hydroxy, CF₃ ou méthoxy,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

6. Composés de formule générale 1 selon l'une des revendications 1 à 5, dans laquelle X⁻ représente bromure ou méthanesulfonate, le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

7. Composés de formule générale 1 selon l'une des revendications 1 à 6, dans laquelle R¹ et R² représentent méthyle, le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

8. Composés de formule générale 1 selon l'une des revendications 1 à 7, dans laquelle R³ représente hydrogène ou méthyle, le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

9. Composés de formule générale 1 selon l'une des revendications 1 à 8, dans laquelle R⁴ représente biphényle, benzhydryle, fluorènyle ou biphénylméthyle, le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide, solvates et hydrates pharmacologiquement acceptables.

10. Composé de formule générale 1 selon l'une des revendications 1 à 9 comme médicament.

11. Utilisation d'un composé selon la formule générale 1 selon l'une des revendications 1 à 9 pour la fabrication d'un médicament destiné au traitement de l'asthme, de la COPD, des bradycardies sinusales vagales, des troubles du rythme cardiaque, des spasmes dans le tractus gastrointestinal, des spasmes dans les voies conductrices d'urée et les troubles de la menstruation.

12. Compositions pharmaceutiques, contenant comme principe actif un ou plusieurs composés de formule générale 1 selon l'une des revendications 1 à 9 ou leurs sels physiologiquement acceptables, le cas échéant en combinaison avec des auxiliaires et/ou véhicules usuels.

13. Compositions pharmaceutiques selon la revendication 11, **caractérisées en ce que** celles-ci contiennent, outre un ou plusieurs des composés de formule 1, également au moins un autre principe actif, qui est choisi dans le groupe constitué par les bêta-mimétiques, les anti-allergiques, les antagonistes de PAF, les inhibiteurs de PDE IV, les antagonistes de leucotriènes, les inhibiteurs de p38 kinase, les inhibiteurs de EGFR kinase et les corticostéroïdes.

14. Produits intermédiaires de formule 4 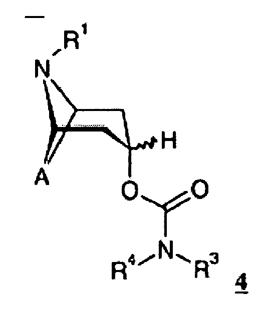 dans laquelle
A représente un radical à doubles liaisons, choisi dans le groupe constitué de : 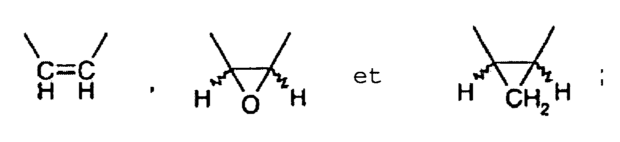
R¹ représente un radical méthyle ou éthyle, qui peut être le cas échéant substitué par cyclopropyle, hydroxy ou fluor, ou
R¹ représente un (alkylène en C₃ à C₄)-X, où X représente chlorure, bromure, méthanesulfonate et p-toluènesulfonate ;
R³ représente un hydrogène ou alkyle en C₁ à C₃, qui peut être le cas échéant substitué par un radical choisi dans le groupe constitué par hydroxy, fluor ou CF₃ ;
R⁴ représente alkyle en C₁ à C₃, qui peut être le cas échéant substitué par un radical choisi dans le groupe constitué par hydroxy, fluor ou CF₃, ou
R⁴ représente un radical phényle, qui est substitué par phényle, qui peut être le cas échéant mono- ou disubstitué par méthyle, fluor, hydroxy ou CF₃, ou
R⁴ représente un radical benzyle, qui peut être le cas échéant substitué sur le cycle phényle par un ou deux radicaux, de préférence un radical choisi dans le groupe constitué par méthyle, éthyle, hydroxy, fluor, CF₃ et phényle, ou
R⁴ représente un radical benzyle, qui peut être le cas échéant substitué sur le pont méthylène par phényle, ou
R⁴ représente un radical de formule 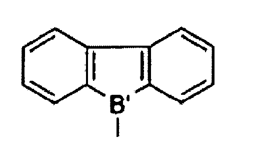 dans laquelle B' représente CH, qui peut être le cas échéant mono- ou disubstitué par un ou plusieurs radicaux choisis dans le groupe constitué par méthyle, fluor, chlore, brome, hydroxy, CF₃ ou méthoxy,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide.

15. Produits intermédiaires de formule 4 selon la revendication 14,
dans laquelle
A représente un radical à doubles liaisons, choisi dans le groupe constitué de : 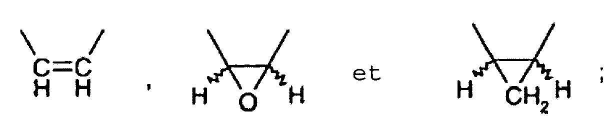
R¹ représente méthyle ;
R³ représente hydrogène ou méthyle ;
R⁴ représente biphényle, benzhydryle, fluorènyle ou biphénylméthyle,
le cas échéant sous la forme des énantiomères ou diastéréomères individuels, des mélanges des énantiomères ou diastéréomères respectifs et le cas échéant sous leurs formes racémiques, et le cas échéant sous la forme de leurs sels d'addition d'acide.
